# EUROPEAN PATENT APPLICATION

(11) **EP 4 717 711 A1**
(43) Date of publication of application: **01.04.2026**
(21) Application number: 24830659.9
(22) Date of filing: 21.06.2024
(51) Int. Cl.: C07K 16/28, C07K 16/46, G01N 33/577, A61K 39/395, A61P 37/02, A61P 35/00

(54) **ANTI-CD3 ANTIBODY AND USE THEREOF**

(30) Priority: 25.06.2023 CN 202310760374
(71) Applicant: Hefei TG Immunopharma Co., Ltd., Hefei, Anhui 230001 (CN)
(72) Inventor: CAO, Guoshuai, Hefei, Anhui 230001 (CN); CHENG, Ying, Hefei, Anhui 230001 (CN); LI, Yangyang, Hefei, Anhui 230001 (CN); WU, Yuwei, Hefei, Anhui 230001 (CN)
(74) Representative: Studio Torta S.p.A.
(86) International application number: PCT/CN2024/100786
(87) International publication number: WO 2025/002014

(57) **Abstract**

Provided are an anti-CD3 antibody or an antigen binding fragment thereof and a use thereof. The anti-CD3 antibody includes heavy chain variable region CDRs and light chain variable region CDRs, the heavy chain variable region CDRs having amino acid sequences as set forth in any one of SEQ ID NOs: 1 to 3, 7 to 8, and 74 to 86, or a conservative modification form thereof; and/or the light chain variable region CDRs having amino acid sequences as set forth in any one of SEQ ID NOs: 4 to 6, 9, and 87 to 92, or a conservative modification form thereof. The provided anti-CD3 antibody or antigen binding fragment thereof can bind to human CD3 protein and reduce the production of pro-inflammatory cytokines. The bispecific antibody constructed using the same has high PBMC-mediated tumor-killing activity and weak ability of promoting the secretion of pro-inflammatory cytokines. A pharmaceutical composition prepared using the anti-CD3 antibody or the antigen-binding fragment thereof or a multi-specific antibody thereof has better safety and has high value for clinical applications and drug development.

## Description

### FIELD

The present disclosure relates to the field of biomedicine, and more particularly, to an anti-CD3 antibody or antigen binding fragment and use thereof.

### BACKGROUND

Cancer is a major disease that affects human survival and development. According to the latest data, there are about 19 million new cancer cases and around 10 million cancer-related deaths worldwide each year, with the incidence and mortality rates showing an upward trend. In addition to surgical resection, traditional cancer treatment methods such as chemotherapy and radiotherapy have serious side effects and are prone to recurrence. In recent years, immunotherapy, including tumor-targeted antibodies, immune checkpoint antibodies, and bispecific antibodies, etc., has become a new hotspot and new hope in cancer treatment. Immunotherapy medicaments, including monoclonal antibodies and bispecific antibodies, mainly achieve anti-cancer effects by promoting the function of autoimmune cells. They have the characteristics of mild side effects and long survival for benefited patients. The immune checkpoint antibodies that have been approved for marketing include PD1-1/L1, CTLA-4, and LAG-3, while the bispecific anti-cancer antibodies include Blinatumomab (CD3×CD19), Kimtrak (CD3×GP100), Mosunetuzumab (CD3×CD20), Tecvayli (CD3×BCMA), etc. The bispecific antibodies have made great progress in the treatment of hematological malignancies. They target CD19, CD20, and BCMA, and can promote T cells to eliminate tumor cells, bringing benefits to patients.

CD3-based bispecific antibodies (hereinafter referred to as "CD3 bispecific antibodies") can promote T cell activation, and kill tumors by recruiting T cells to reach the tumor site, and bridging T cells with tumors. This type of bispecific antibody does not require neoantigens and can guide T cells to kill "cold tumors". After binding to T cells and tumor cells, the CD3 bispecific antibodies trigger strong activation signals. Therefore, the CD3 bispecific antibodies can "ignore" the inhibitory signals of immune checkpoint molecules to a certain extent. However, the CD3 bispecific antibodies can also promote the production of a large number of pro-inflammatory cytokines, such as TNFα, and IL-6, triggering a strong cytokine storm and excessive immune response, which cause damage to the body, and in severe cases, may be life-threatening. Therefore, the CD3-based bispecific antibodies have a good clinical application prospect, but their safety needs to be further improved.

One way to address the safety issues of the CD3 bispecific antibodies is to increase the affinity of the CD3 bispecific antibodies for binding to tumor targets while reducing their affinity for CD3. This enables the CD3 bispecific antibody drugs to be preferentially accumulated at the tumor site, increasing local drug concentrations at the tumor site, reducing peripheral drug concentration, reducing off-target toxicity, lowering the production level of pro-inflammatory cytokines, and reducing on-target toxicity. Therefore, there is an urgent need to develop anti-CD3 antibodies that can bind to human CD3 protein with low affinity, so as to obtain CD3 antibody drugs with better safety and high value in clinical application and drug development.

### SUMMARY

The present disclosure aims to solve at least one of the technical problems existing in the prior art at least to some extent. To this end, one object of the present disclosure is to provide an anti-CD3 antibody or antigen binding fragment thereof. Another object of the present disclosure is to provide a multi-specific antibody constructed using the anti-CD3 antibody or the antigen binding fragment thereof. The anti-CD3 antibody or antigen binding fragment thereof of the present disclosure can bind to human CD3 protein and reduce the production of pro-inflammatory cytokines. The bispecific antibody constructed using the anti-CD3 antibody or antigen binding fragment thereof has high PBMC-mediated tumor-killing activity and weak ability of promoting the secretion of pro-inflammatory cytokines. Therefore, the pharmaceutical composition prepared using the anti-CD3 antibody or antigen binding fragment thereof or multi-specific antibody thereof has better safety and high value in clinical application and drug development.

The present disclosure is made based on the inventor's discoveries and understandings of the following facts and problems.

Currently, the most commonly used CD3 antibodies among bispecific antibodies are TR66 and UCHT1. On the one hand, neither of these two antibodies binds to monkey CD3, which increases the difficulty of non-clinical evaluation of the medicament; on the other hand, these two antibodies bind very strongly to T cells, which increases the safety risk of CD3 bispecific antibody drugs constructed based thereon. The inventors performed affinity engineering on Cross3, an anti-CD3 antibody with high affinity for CD3 (KD=0.165nM) and cross-reactivity to humans and monkeys. Through extensive screening, a series of anti-CD3 antibodies that can bind to human CD3 protein with low affinity have been obtained. Furthermore, based on this series of anti-CD3 antibodies, CD3 bispecific antibodies have been constructed, and 19 anti-CD3 antibodies have been screened and obtained. Compared with Cross3, the specific recognition and binding of bispecific antibodies to tumor antigens are not affected by the CD3 antigen binding region. Further experimental results demonstrated that: 1) the CD3 bispecific antibodies of the present disclosure bind to CD3E&D proteins and specifically bind to tumor antigens (such as PDL1 and Trop2), and their tumor antigen binding activity is substantially equivalent to that of a bispecific antibody constructed based on Cross3; 2) cellular level experimental results showed that the CD3 bispecific antibodies of the present disclosure can bind to T cells and specifically bind to tumor cells (such as A-375-Trop2 cells, A-375 melanoma cells, HCT-15 colorectal cancer cells, and A549 lung cancer cells), and its tumor cell binding ability is substantially equivalent to that of a bispecific antibody constructed based on Cross3 antibody; 3) experimental results of promoting the secretion of cytokines by PBMCs showed that the CD3 bispecific antibodies of the present disclosure has reduced ability of promoting the secretion of pro-inflammatory cytokines.

The inventors further verified the tumor cell killing activity of the above-described CD3 bispecific antibodies through PBMCs tumor cell killing assays. The results showed that the bispecific antibodies (such as Cross313×Trop2, Cross316×Trop2, Cross325×Trop2, Cross313×PDL1, Cross316×PDL1, and Cross325×PDL1) constructed based on the anti-CD3 antibodies Cross313, Cross316, and Cross325 of the present disclosure can achieve the highest killing effect or can promote 100% tumor cell killing by PBMCs, and have comparable tumor cell killing effects to the bispecific antibodies (such as Cross3×Trop2 and Cross3×PDL1) constructed based on the anti-CD3 antibody Cross3.

The above experimental results showed that the CD3 bispecific antibody developed based on the anti-CD3 antibody or antigen binding fragment thereof of the present disclosure has high PBMC-mediated tumor-killing activity and weak ability of promoting the secretion of pro-inflammatory cytokines, better safety, and high value in clinical application and drug development.

Therefore, in a first aspect of the present disclosure, the present disclosure provides an anti-CD3 antibody or antigen binding fragment thereof. According to an embodiment of the present disclosure, the anti-CD3 antibody or antigen binding fragment thereof includes heavy chain variable region CDRs and light chain variable region CDRs, the heavy chain variable region CDRs have amino acid sequences as set forth in any one of SEQ ID NOs: 1 to 3, 7 to 8, and 74 to 86, or a conservative modification form thereof; and/or the light chain variable region CDRs have amino acid sequences as set forth in any one of SEQ ID NOs: 4 to 6, 9, and 87 to 92, or a conservative modification form thereof.

The anti-CD3 antibody or antigen binding fragment thereof according to the embodiments of the present disclosure can bind to human CD3 protein and reduce the production of pro-inflammatory cytokines. The CD3 bispecific antibody developed based on this CD3 antibody or the antigen binding fragment thereof has high PBMC-mediated tumor-killing activity and weak ability of promoting the secretion of pro-inflammatory cytokines. After being prepared into a medicament, on the one hand, the anti-CD3 antibody or antigen binding fragment thereof can be preferentially accumulated at the tumor site, increasing the local drug concentration at the tumor site and reducing the peripheral drug concentration, thereby reducing the risk of off-target toxicity. On the other hand, this bispecific antibody medicament can reduce the production of pro-inflammatory cytokines and reduce the risk of on-target toxicity. Furthermore, the drugs prepared using the anti-CD3 antibody or antigen binding fragment thereof can be used to prevent and/or treat a CD3-related disease. CD3 bispecific antibody drugs developed based on the anti-CD3 antibody or antigen binding fragment thereof are safer and can be used to prevent and/or treat a cancer or tumor or infection or autoimmune disease. Therefore, the anti-CD3 antibody or antigen binding fragment thereof of the present disclosure has good value in clinical application and drug development.

In a second aspect, the present disclosure provides a multi-specific antibody. According to an embodiment of the present disclosure, the multi-specific antibody includes a first antigen binding region and a second antigen binding region. The first antigen binding region includes the anti-CD3 antibody or antigen binding fragment thereof according to the first aspect of the present disclosure; and the second antigen binding region has a biomolecule binding activity, the biomolecule not being CD3.

As described above, the multi-specific antibody (such as bispecific antibody) developed based on the anti-CD3 antibody or antigen binding fragment thereof according to the first aspect of the present disclosure can be preferentially accumulated at the tumor site after being prepared into medicaments, increasing the local drug concentration at the tumor site and reducing the peripheral drug concentration, thereby reducing the risk of off-target toxicity. At the same time, the production of pro-inflammatory cytokines can be reduced, and the risk of on-target toxicity of multi-specific antibodies (such as bispecific antibodies) can be reduced. Therefore, the multi-specific antibody based on the anti-CD3 antibody or antigen binding fragment thereof of the first aspect of the present disclosure has better safety and high value in clinical application and drug development.

In a third aspect of the present disclosure, the present disclosure provides a conjugate. According to an embodiment of the present disclosure, the conjugate includes: the antibody or antigen binding fragment thereof according to the first aspect of the present disclosure or the multi-specific antibody according to the second aspect of the present disclosure.

As described above, the anti-CD3 antibody or antigen binding fragment thereof according to the embodiments of the present disclosure can be used to prepare bispecific antibody drugs. On the one hand, the prepared bispecific antibody drugs can be preferentially accumulated at the tumor site, increasing the local drug concentration at the tumor site and reducing the peripheral drug concentration, thereby reducing the risk of off-target toxicity. On the other hand, the bispecific antibody can reduce the production of pro-inflammatory cytokines, reducing the risk of on-target toxicity of multi-specific antibodies (such as bispecific antibodies). Furthermore, the drugs prepared using the above-described anti-CD3 antibody or antigen binding fragment thereof can be used to prevent and/or treat a CD3-related disease. The CD3 bispecific antibody drugs developed based on the anti-CD3 antibody or antigen binding fragment thereof are safer and can be used to prevent and/or treat a cancer or tumor or infection or autoimmune disease. Therefore, the conjugate including the anti-CD3 antibody or antigen binding fragment thereof according to the first aspect of the present disclosure and the multi-specific antibody according to the second aspect of the present disclosure has good clinical application value and drug development value.

In a fourth aspect, the present disclosure provides a nucleic acid. According to an embodiment of the present disclosure, the nucleic acid encodes the anti-CD3 antibody or antigen binding fragment thereof according to the first aspect of the present disclosure or the multi-specific antibody according to the second aspect of the present disclosure. According to an embodiment of the present disclosure, the antibody or antigen binding fragment thereof of the first aspect of the present disclosure or the multi-specific antibody of the second aspect of the present disclosure encoded by the nucleic acid is suitable for developing monoclonal antibody or bispecific antibody drugs with higher safety. Furthermore, the protein encoded by the nucleic acid can be used to prevent and/or treat a CD3-related disease or prevent and/or treat a cancer or tumor or infection or autoimmune disease.

In a fifth aspect of the present disclosure, the present disclosure provides a vector or transformant. According to an embodiment of the present disclosure, the vector or transformant contains the nucleic acid according to the fourth aspect of the present disclosure. Thus, with the constructed vector or transformant, it is feasible to effectively express the antibody or antigen binding fragment thereof of the first aspect of the present disclosure or the multi-specific antibody of the second aspect of the present disclosure, and to obtain the conjugate of the third aspect of the present disclosure.

In a sixth aspect, the present disclosure provides a cell. According to an embodiment of the present disclosure, the cell carries the nucleic acid according to the fourth aspect of the present disclosure or the vector or transformant according to the fifth aspect of the present disclosure, or expresses the antibody or antigen binding fragment thereof according to the first aspect of the present disclosure or the multi-specific antibody of the second aspect of the present disclosure. According to an embodiment of the present disclosure, the cells can efficiently express the antibody or antigen binding fragment thereof of the first aspect of the present disclosure or the multi-specific antibody of the second aspect of the present disclosure under suitable conditions. Furthermore, an antibody or antigen binding fragment thereof or multi-specific antibody thereof with improved safety that can bind to human CD3 protein can be obtained.

In a seventh aspect of the present disclosure, the present disclosure provides a pharmaceutical composition. According to an embodiment of the present disclosure, the pharmaceutical composition includes: the antibody or antigen binding fragment thereof of the first aspect of the present disclosure, the multi-specific antibody of the second aspect of the present disclosure, the conjugate of the third aspect of the present disclosure, the nucleic acid of the fourth aspect of the present disclosure, or the vector or transformant of the fifth aspect of the present disclosure. Thus, the obtained medicament can be further used for preventing and/or treating a CD3-related disease, or further used for preventing and/or treating a CD3-related disease and/or cancer or tumor or infection or autoimmune disease.

In an eighth aspect of the present disclosure, the present disclosure provides a kit. According to an embodiment of the present disclosure, the kit includes the antibody or antigen binding fragment thereof according to the first aspect of the present disclosure or the multi-specific antibody according to the second aspect of the present disclosure. Thus, the obtained kit can be used for a CD3-related research.

In a ninth aspect of the present disclosure, the present disclosure provides a use of the antibody or antigen binding fragment thereof according to the first aspect of the present disclosure or the multi-specific antibody according to the second aspect of the present disclosure in the manufacture of a kit for detecting CD3. Those skilled in the art will appreciate that the above-described features and advantages for the antibody or antigen binding fragment thereof are also applicable to this use and will not be reiterated here.

In a tenth aspect of the present disclosure, the present disclosure provides a use of the antibody or antigen binding fragment thereof of the first aspect of the present disclosure, the multi-specific antibody of the second aspect of the present disclosure, the conjugate of the third aspect of the present disclosure, the nucleic acid of the fourth aspect of the present disclosure, the vector or transformant of the fifth aspect of the present disclosure, or the pharmaceutical composition of the seventh aspect of the present disclosure in the manufacture of a medicament for preventing and/or treating a CD3-related disease and/or cancer or tumor or infection or autoimmune disease. The antibody or antigen binding fragment thereof, the multi-specific antibody, the conjugate, the nucleic acid, the vector or transformant, or the pharmaceutical composition of the present disclosure can be further prepared into medicaments, which can be used clinically to prevent or treat a disease.

Those skilled in the art will appreciate that the features and advantages described above for the antibody or antigen binding fragment thereof, the conjugate, the nucleic acid, the vector or transformant, or the pharmaceutical composition are also applicable to this use and will not be reiterated here.

### Beneficial effects:

(1) The anti-CD3 antibodies Cross303, Cross307, Cross308, Cross309, Cross310, Cross311, Cross312, Cross313, Cross314, Cross316, Cross317, Cross318, Cross323, Cross325, Cross326, Cross3ZH07, Cross3ZH09, Cross3ZH16, and Cross3ZH23 obtained in the present disclosure can reduce the production of pro-inflammatory cytokines caused by the CD3 antigen binding moiety compared with the parental antibody Cross3.

(2) The bispecific antibody constructed from the antibodies Cross313, Cross316, and Cross325 obtained in the present disclosure have weaker T cell binding ability compared with the bispecific antibody constructed from the parental antibody Cross3.

(3) The bispecific antibody constructed from the antibodies Cross313, Cross316, and Cross325 obtained in the present disclosure can also promote PBMC to kill tumor cells to the greatest extent and has weaker performance in promoting the production of pro-inflammatory cytokines and better safety compared with the bispecific antibody constructed from the parental antibody Cross3,

Additional aspects and advantages of the present disclosure will be provided at least in part in the following description, or will become apparent at least in part from the following description, or can be learned from practicing of the present disclosure.

### BRIEF DESCRIPTION OF THE DRAWINGS

The above and/or additional aspects and advantages of the present disclosure will become apparent and easily understandable from the following description of embodiments in conjunction with the accompanying drawings, in which:
FIG. 1 is a comparison of amino acid mutations between an affinity-reduced antibody and a parental antibody Cross3 according to an embodiment of the present disclosure.
FIG. 2 is a graph showing the SPR results of affinity assay between a parental antibody Cross3 as well as an affinity-reduced antibody and CD3E&D protein according to an embodiment of the present disclosure.
FIG. 3 is a graph showing the ELISA results of the binding of a parental antibody Cross3 as well as an affinity-reduced antibody to CD3E&D protein according to an embodiment of the present disclosure.
FIG. 4 is a graph showing the flow cytometry results of the binding of a parental antibody Cross3 as well as an affinity-reduced antibody to CD8 T cells according to an embodiment of the present disclosure.
FIG. 5 is a schematic diagram of the formats of CD3×Trop2 and CD3×PDL1 bispecific antibody constructed from a parental antibody Cross3 and an affinity-reduced antibody according to an embodiment of the present disclosure;
FIG. 6 is a graph showing the ELISA results of the binding of a CD3×Trop2 bispecific antibody constructed from a parental antibody Cross3 and an affinity-reduced antibody to CD3E&D protein according to an embodiment of the present disclosure.
FIG. 7 is a graph showing the ELISA results of the binding of a CD3×Trop2 bispecific antibody constructed from a parental antibody Cross3 and an affinity-reduced antibody to Trop2 protein according to an embodiment of the present disclosure.
FIG. 8 is a graph showing the ELISA results of a CD3×Trop2 bispecific antibody constructed from a parental antibody Cross3 and an affinity-reduced antibody bridging CD3E&D and Trop2 proteins according to an embodiment of the present disclosure.
FIG. 9 is a graph showing the flow cytometry results of the binding of a CD3×Trop2 bispecific antibody constructed from a parental antibody Cross3 and an affinity-reduced antibody to CD8 T cells according to an embodiment of the present disclosure.
FIG. 10 is a graph showing the flow cytometry results of the binding of a CD3×Trop2 bispecific antibody constructed from a parental antibody Cross3 and an affinity-reduced antibody to A-375-Trop2 melanoma cells according to an embodiment of the present disclosure.
FIG. 11 is a graph showing the results of a CD3×Trop2 bispecific antibody constructed from a parental antibody Cross3 and an affinity-reduced antibody promoting PBMC to kill A-375-Trop2 melanoma cells according to an embodiment of the present disclosure.
FIG. 12 is a graph showing the results of Cross3×Trop2, Cross313×Trop2, Cross316×Trop2, and Cross325×Trop2 bispecific antibodies promoting PBMC to kill A-375-Trop2 melanoma cells according to an embodiment of the present disclosure.
FIG. 13 is a graph showing the results of Cross3×Trop2, Cross313×Trop2, Cross316×Trop2, and Cross325×Trop2 bispecific antibodies promoting the secretion of pro-inflammatory cytokines by PBMC according to an embodiment of the present disclosure.
FIG. 14 is a graph showing the ELISA results of the binding of a CD3×PDL1 bispecific antibody constructed from a parental antibody Cross3 and an affinity-reduced antibody to CD3E&D protein according to an embodiment of the present disclosure.
FIG. 15 is a graph showing the ELISA results of the binding of a CD3×PDL1 bispecific antibody constructed from a parental antibody Cross3 and an affinity-reduced antibody to PDL1 protein according to an embodiment of the present disclosure.
FIG. 16 is a graph showing the flow cytometry results of the binding of a CD3×PDL1 bispecific antibody constructed from a parental antibody Cross3 and an affinity-reduced antibody to CD8 T cells according to an embodiment of the present disclosure.

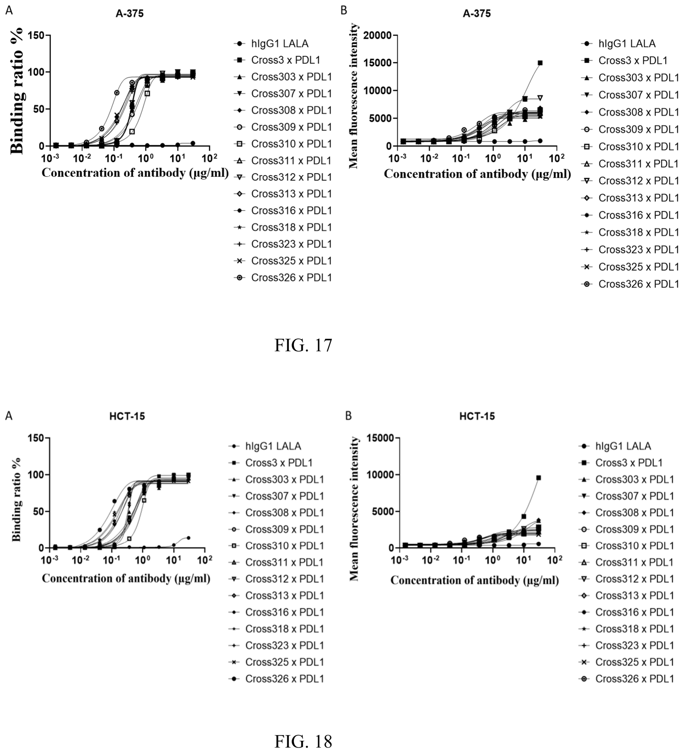

FIG. 17 is a graph showing the flow cytometry results of the binding of a CD3×PDL1 bispecific antibody constructed from a parental antibody Cross3 and an affinity-reduced antibody to A-375 melanoma cells according to an embodiment of the present disclosure.
FIG. 18 is a graph showing the flow cytometry results of the binding of a CD3×PDL1 bispecific antibody constructed from a parental antibody Cross3 and an affinity-reduced antibody to HCT-15 colorectal cancer cells according to an embodiment of the present disclosure.
FIG. 19 is a graph showing the flow cytometry results of the binding of a CD3×PDL1 bispecific antibody constructed from a parental antibody Cross3 and an affinity-reduced antibody to A-549 lung cancer cells according to an embodiment of the present disclosure.
FIG. 20 is a graph showing the results of a CD3×PDL1 bispecific antibody constructed from a parental antibody Cross3 and an affinity-reduced antibody promoting PBMC to kill A-375 melanoma cells according to an embodiment of the present disclosure.
FIG. 21 is a graph showing the results of Cross3×PDL1, Cross313×PDL1, Cross316×PDL1, and Cross325×PDL1 promoting PBMC to kill A-375 melanoma cells according to an embodiment of the present disclosure.
FIG. 22 is a graph showing the results of a CD3×PDL1 bispecific antibody constructed from a parental antibody Cross3 and an affinity-reduced antibody promoting PBMC to kill HCT-15 colorectal cancer cells according to an embodiment of the present disclosure.
FIG. 23 is a graph showing the results of a CD3×PDL1 bispecific antibody constructed from a parental antibody Cross3 and an affinity-reduced antibody promoting the secretion of pro-inflammatory cytokines by PBMC after co-incubation with A-375 according to an embodiment of the present disclosure; and
FIG. 24 is a graph showing the results of a CD3×PDL1 bispecific antibody constructed from a parental antibody Cross3 and an affinity-reduced antibody promoting the secretion of pro-inflammatory cytokines by PBMC after co-incubation with HCT-15 according to an embodiment of the present disclosure.

### DETAILED DESCRIPTION

Embodiments of the present disclosure will now be described in detail, examples of which are illustrated in the accompanying drawings. The embodiments described below with reference to the accompanying drawings are exemplary and are intended to explain the present disclosure, but should not be construed as limiting the present disclosure.

**In** addition, terms "first" and "second" are only used for descriptive purposes, and cannot be understood as indicating or implying relative importance, or implicitly indicating the number of indicated technical features. Therefore, the features defined with "first" and "second" may explicitly or implicitly include at least one of the features. In the description of the present disclosure, "plurality/more" means at least two, such as two or three, unless otherwise explicitly specified.

The endpoint values or any values of the ranges disclosed herein are not limited to the precise ranges or values. These ranges or values should be understood to include values approximating such ranges or values. For numerical ranges, one or more new numerical ranges can be obtained by combining the endpoint values of the respective ranges, an endpoint value and an individual point value within the respective ranges, and individual point values within the respective ranges with each other, and these numerical ranges should be regarded as specifically disclosed herein.

### Terms and definitions

**In** order to make the present disclosure more easily understood, certain technical and scientific terms are specifically defined below. Unless obviously and clearly defined elsewhere in this document, all other technical and scientific terms used herein have the meanings commonly understood by one of ordinary skill in the art to which the present disclosure belongs. Abbreviations for amino acid residues are standard 3-letter and/or 1-letter codes used in the art referring to one of the 20 commonly used L-amino acids.

As used herein, the term "antibody" generally refers to an antibody that can recognize one or more antigenic epitopes, including but not limited to monoclonal antibodies, polyclonal antibodies, dimers, multimers, multi-specific antibodies (e.g., bispecific antibodies), heavy-chain-only antibodies, triple-chain antibodies, single-chain Fv (scFv), nanobodies, etc., and also includes antibody fragments, as long as they exhibit the desired biological activity. The antibody can be murine, human, humanized, chimeric, or from other species. The antibody can refer to a full-length heavy chain, a full-length light chain, an intact immunoglobulin molecule; or an immunologically active portion of any one of these polypeptides, i.e., a molecule or portion thereof that contains an antigen-binding site that immunospecifically binds to a target antigen of interest, and such targets include but are not limited to cancer cells or cells that produce autoimmune antibodies associated with an autoimmune disease.

As used herein, in the variable region, the amino acid composition and arrangement order of some regions have a higher degree of variation, which is called the hypervariable region (HVR). The hypervariable region is the location where the antigen and antibody bind, so it is also called the complementarity-determining region (CDR). There are three CDR regions in both the heavy chain variable region and the light chain variable region. For example, it generally includes: amino acid residues near 23-34 (L1), 50-56 (L2), and 89-97 (L3) in the light chain variable region, and near 31-35B (H1), 50-65 (H2), and 95-102 (H3) in the heavy chain variable region; and/or amino acid residues from a "hypervariable loop", such as amino acid residues near 26-32 (L1), 50-52 (L2), and 91-96 (L3) in the light chain variable region and near 26-32 (H1), 53-55 (H2), and 96-101 (H3) in the heavy chain variable region.

As used herein, the term "anti-CD3 antibody" refers to an antibody capable of binding to CD3. Such antibodies are also referred to herein as "CD3-binding antibody".

As used herein, the term "Fab-Linker" refers to a polypeptide segment obtained by linking Fab VH-CH1 and Fab VL-CL of an antibody Fab fragment via a linking peptide, including but not limited to: scFab, scFab△ (the linking peptide contains no disulfide bond).

As used herein, the term "antigen binding fragment" is equivalent to "antibody fragment" or "antigen binding antibody fragment", and may include a portion, generally the binding region or variable region, of an intact antibody, including but is not limited to, Fv, scFv, Fab, Fab', Fab'-SH, F(ab')₂, scFv-Fc fragments, or bispecific antibodies (BsAbs), linear antibodies, or any fragment that can increase half-life by chemical modification, such as the addition of poly(alkylene) glycols, such as polyethylene glycol ("PEGylation") (PEGylated fragments referred to as Fv-PEG, scFv-PEG, Fab-PEG, F(ab')₂-PEG, or Fab'-PEG) ("PEG" is polyethylene glycol) or by incorporation into liposomes.

As used herein, the term "chimeric antibody" refers to a recombinant antibody obtained by replacing the amino acid sequence of the constant region of a monoclonal antibody from one species (e.g. mouse) with the constant region of an antibody from another species (e.g. human) using recombinant DNA technology.

As used herein, the term "humanized antibody" refers to a recombinant antibody obtained by completely replacing the amino acid sequences of the constant region and the non-CDR (Fv framework region (FR)) in the variable region of a monoclonal antibody from one species (e.g. mouse) with the amino acid sequences of the constant region and the non-CDR in the variable region of an antibody from another species (e.g. human) using recombinant DNA technology. That is, when the constant region of an antibody is humanized, it is referred to as a chimeric antibody, and when the amino acid sequences of the constant region and the non-CDR in variable region are all humanized, it is referred to as a humanized antibody. Methods of humanization may be carried out with reference to conventional antibody engineering techniques and will not be reiterated herein.

With respect to nucleotides, the terms "homology," "identity," or "similarity" are used to describe or compare the degree of nucleotide similarity between two or more nucleotide sequences. The percentage of "sequence homology" between a first sequence and a second sequence can be calculated by dividing [the number of identical nucleotides in the first sequence that are identical to the nucleotides at corresponding positions]. [nucleotides in the second sequence] minus [the total number of nucleotides in the first sequence] and then multiplied by [100%], where each deletion, insertion, substitution, or addition of a nucleotide relative to the first nucleotide sequence in the second nucleotide sequence is considered a difference at a single nucleotide (position). Alternatively, the degree of sequence identity between two or more nucleotide sequences can be calculated using known computer algorithms for sequence alignment, such as NCBI Blast v2.0, using standard settings. Some other techniques, computer algorithms, and setups for determining the degree of sequence identity are described, for example, in WO 04/037999, EP 0 967 284, EP 1 085 089, WO 00/55318, WO 00/78972, WO 98/49185, and GB 2357768-A.

With respect to polypeptides, the terms "(substantial) homology", "identity", or "similarity" are used to describe or compare the degree of amino acid similarity between two or more polypeptides or designated sequences thereof at optimal alignment and comparison (with appropriate insertions or deletions of nucleotides). The % homology between two sequences varies with the number of identical positions shared by the sequences at optimal alignment (i.e., % homology = number of identical positions/total number of positions × 100), where optimal alignment is determined by taking into account the number of gaps that need to be introduced, and the length of each gap, to achieve an optimal alignment of the two sequences. The comparison of two sequences and determination of identity percentage between two sequences can be accomplished using a mathematical algorithm, as described in the nonlimiting examples below.

As used herein, the term "an amino acid sequence in conservative modification form" refers to an amino acid modification that does not significantly affect or alter the binding characteristics of the antibody including the amino acid sequence, and the modification includes the substitutions, additions and deletions of the amino acids. Modifications can be introduced into the antibodies of the present disclosure by standard techniques such as site-directed mutagenesis and PCR-mediated mutagenesis. Conservative amino acid substitution refers to the substitution in which the amino acid residues are replaced by amino acid residues having similar side chains. Families of amino acid residues having similar side chains have been identified in the art. These families include amino acids with basic side chains, e.g. lysine, arginine, and histidine; amino acids with acidic side chains, e.g. aspartic acid, and glutamic acid; amino acids with uncharged polar side chains, e.g. glycine, asparagine, glutamine, serine, threonine, tyrosine, cysteine, and tryptophan; amino acids with nonpolar side chains, e.g. alanine, valine, leucine, isoleucine, proline, phenylalanine, and methionine; amino acids with β-branched side chains, e.g. threonine, valine, and isoleucine, and amino acids with aromatic side chains, e.g. tyrosine, phenylalanine, tryptophan, and histidine.

As used herein, the term "conjugate" is understood in context to refer to an antibody or antigen binding fragment thereof conjugated to a coupling moiety such as a vector substance, medicament, toxin, cytokine, protein tag, modifier, therapeutic agent, chemotherapeutic agent, etc., using any covalent or non-covalent bioconjugation strategy.

As used herein, the term "vector" generally refers to a nucleic acid molecule capable of being inserted into a suitable host for self-replication, which transfers the inserted nucleic acid molecule into and/or between host cells. The vector may include a vector primarily used to insert DNA or RNA into cells, a vector primarily used to replicate DNA or RNA, and a vector primarily used for expression by transcription and/or translation of DNA or RNA. The vector also includes a vector having multiple functions as described above. The vector may be a polynucleotide that can be transcribed and translated into a polypeptide when introduced into a suitable host cell. Typically, by culturing appropriate host cells containing the vector, the vector can produce the desired expression product.

As used herein, the term "pharmaceutical composition" generally refers to unit dosage form, and may be prepared by any of the methods well known in the art of pharmacy. All methods include the step of combining the active ingredient with the carrier which constitutes one or more accessory ingredients. In general, the compositions are prepared by uniformly and thoroughly combining the active compound with liquid carriers, finely divided solid carriers, or both.

As used herein, the term "pharmaceutically acceptable" refers to substances that are suitable for use in humans and/or mammals without excessive adverse side effects (such as toxicity, irritation, and allergic response), i.e., a substance with at a reasonable benefit/risk ratio.

As used herein, the term "pharmaceutically acceptable adjuvant" may include any solvent, solid excipient, diluent, or other liquid excipient, etc., suitable for a particular target dosage form. Except for any conventional adjuvants that are incompatible with the compounds of the present disclosure, for example which produce any adverse biological effects or interact in a deleterious manner with any other component of the pharmaceutically acceptable composition, their use is contemplated by the present disclosure.

As used herein, the term "administering/administration" refers to introducing a predetermined amount of a substance into a patient in a suitable manner. The antibody or antigen binding fragment, recombinant protein, multi-specific antibody, conjugate, or pharmaceutical composition of the present disclosure can be administered via any common route as long as it can reach the intended tissue. Various routes of administration are contemplated, including peritoneal, intravenous, intramuscular, subcutaneous injection, and the like, but the present disclosure is not limited to these exemplified routes of administration. Preferably, the composition of the present disclosure is administered by intravenous injection or subcutaneous injection.

As used herein, the term "treat/treatment" refers to the treatment intended to achieve a desired pharmacological and/or physiological effect. The effect may be prophylactic in terms of complete or partial prevention of the disease or its symptoms, and/or therapeutic in terms of partial or complete cure of the disease and/or its adverse effects. As used herein, "treat/treatment" encompasses diseases in mammals, particularly humans, and includes: (a) preventing the occurrence of a disease or condition in an individual who is susceptible to the disease but has not yet been diagnosed with the disease; (b) inhibiting the disease, such as arresting the progression of the disease; or (c) alleviating the disease, such as alleviating the symptoms associated with the disease. As used herein, "treat/treatment" encompasses any administration of a medicament or compound to a subject to treat, cure, alleviate, ameliorate, lessen, or inhibit a disease in the subject, including but not limited to administering a medicament containing the compound according to the present disclosure to a subject in need thereof.

As used herein, the term "effective amount" or "effective dose" refers to an amount that is capable of producing a function or activity in humans and/or animals and is acceptable to humans and/or animals.

The present disclosure provides an anti-CD3 antibody or antigen binding fragment thereof, a multi-specific antibody, a conjugate, a nucleic acid molecule, a vector or transformant, a cell, a pharmaceutical composition, a kit, and uses thereof, which are described in detail below.

### Anti-CD3 antibody or antigen binding fragment thereof

The present disclosure provides an anti-CD3 antibody or antigen binding fragment thereof. The anti-CD3 antibody or antigen binding fragment thereof includes heavy chain variable region CDRs and light chain variable region CDRs. The heavy chain variable region CDRs have each an amino acid sequence as set forth in one of SEQ ID NOs: 1 to 3, 7 to 8, and 74 to 86, or a conservative modification form thereof; and/or the light chain variable region CDRs have each an amino acid sequences as set forth in one of SEQ ID NOs: 4 to 6, 9, and 87 to 92, or a conservative modification form thereof.

The anti-CD3 antibody or antigen binding fragment thereof according to the present disclosure can bind to human CD3 protein and reduce the production of pro-inflammatory cytokines. The CD3 bispecific antibody developed based on this CD3 antibody or its antigen binding fragment has high PBMC-mediated tumor-killing activity and weak ability of promoting the secretion of pro-inflammatory cytokines. After being prepared into a medicament, on the one hand, the anti-CD3 antibody or antigen binding fragment thereof can be preferentially accumulated at the tumor site, increasing the local drug concentration at the tumor site and reducing the peripheral drug concentration, thereby reducing the risk of off-target toxicity. On the other hand, this bispecific antibody medicament can reduce the production of pro-inflammatory cytokines and reduce the risk of on-target toxicity. Furthermore, the drugs prepared using the anti-CD3 antibody or antigen binding fragment thereof can be used to prevent and/or treat a CD3-related disease. CD3 bispecific antibody drugs developed based on the anti-CD3 antibody or antigen binding fragment thereof are safer and can be used to prevent and/or treat a cancer or tumor or infection or autoimmune disease. Therefore, the anti-CD3 antibody or antigen binding fragment thereof of the present disclosure has good value in clinical application and drug development.

It should be noted that one or more amino acid residues in the CDR region of the antibody or antigen binding fragment thereof of the present disclosure can be replaced with other amino acid residues from the same side chain family, and the retained function of the modified antibody can be tested using the functional assay method described herein. Preferably, the number of conservative modifications does not exceed one or two.

According to an embodiment of the present disclosure, the heavy chain variable region CDRs have amino acid sequences as set forth in SEQ ID NOs: 1 to 2 or a conservative modification form thereof, and an amino acid sequence as set forth in one of SEQ ID NOs: 3, 7 to 8, and 74 to 86, or a conservative modification form thereof; and the light chain variable region CDRs have amino acid sequences as set forth in SEQ ID NOs: 4 to 5 or a conservative modification form thereof, and an amino acid sequence as set forth in one of SEQ ID NOs: 6, 9, and 87 to 92, or a conservative modification form thereof.

According to an embodiment of the present disclosure, the antibody or antigen binding fragment thereof includes: heavy chain variable region CDR1 having an amino acid sequence as set forth in SEQ ID NO: 1, heavy chain variable region CDR2 having an amino acid sequence as set forth in SEQ ID NO: 2, heavy chain variable region CDR3 having an amino acid sequence as set forth in one of SEQ ID NO: 3, SEQ ID NOs: 7 to 8, and SEQ ID NOs: 74 to 86; light chain variable region CDR1 having an amino acid sequence as set forth in SEQ ID NO: 4, light chain variable region CDR2 having an amino acid sequence as set forth in SEQ ID NO: 5, light chain variable region CDR3 having an amino acid sequence as set forth in one of SEQ ID NOs: 6, 9, and 87 to 92.

According to an embodiment of the present disclosure, the heavy chain variable region CDR3 has an amino acid sequence as set forth in one of SEQ ID NOs: 3 and 7 to 8; and/or the light chain variable region CDR3 has an amino acid sequence as set forth in SEQ ID NO: 6 or 9.

According to an embodiment of the present disclosure, the antibody or antigen binding fragment thereof includes any one of the following groups: 1) the heavy chain variable region CDR1, the heavy chain variable region CDR2, and the heavy chain variable region CDR3 as set forth in SEQ ID NOs: 1, 2, and 74, respectively, and the light chain variable region CDR1, the light chain variable region CDR2, and the light chain variable region CDR3 as set forth in SEQ ID NOs: 4, 5, and 6, respectively; 2) the heavy chain variable region CDR1, the heavy chain variable region CDR2, and the heavy chain variable region CDR3 as set forth in SEQ ID NOs: 1, 2, and 75, respectively, and the light chain variable region CDR1, the light chain variable region CDR2, and the light chain variable region CDR3 as set forth in SEQ ID NOs: 4, 5, and 6, respectively; 3) the heavy chain variable region CDR1, the heavy chain variable region CDR2, and the heavy chain variable region CDR3 as set forth in SEQ ID NOs: 1, 2, and 76, respectively, and the light chain variable region CDR1, the light chain variable region CDR2, and the light chain variable region CDR3 as set forth in SEQ ID NOs: 4, 5, and 6, respectively; 4) the heavy chain variable region CDR1, the heavy chain variable region CDR2, and the heavy chain variable region CDR3 as set forth in SEQ ID NOs: 1, 2, and 77, respectively, and the light chain variable region CDR1, the light chain variable region CDR2, and the light chain variable region CDR3 as set forth in SEQ ID NOs: 4, 5, and 6, respectively; 5) the heavy chain variable region CDR1, the heavy chain variable region CDR2, and the heavy chain variable region CDR3 as set forth in SEQ ID NOs: 1, 2, and 78, respectively, and the light chain variable region CDR1, the light chain variable region CDR2, and the light chain variable region CDR3 as set forth in SEQ ID NOs: 4, 5, and 6, respectively; 6) the heavy chain variable region CDR1, the heavy chain variable region CDR2, and the heavy chain variable region CDR3 as set forth in SEQ ID NOs: 1, 2, and 79, respectively, and the light chain variable region CDR1, the light chain variable region CDR2, and the light chain variable region CDR3 as set forth in SEQ ID NOs: 4, 5, and 6, respectively; 7) the heavy chain variable region CDR1, the heavy chain variable region CDR2, and the heavy chain variable region CDR3 as set forth in SEQ ID NOs: 1, 2, and 80, respectively, and the light chain variable region CDR1, the light chain variable region CDR2, and the light chain variable region CDR3 as set forth in SEQ ID NOs: 4, 5, and 6, respectively; 8) the heavy chain variable region CDR1, the heavy chain variable region CDR2, and the heavy chain variable region CDR3 as set forth in SEQ ID NOs: 1, 2, and 81, respectively, and the light chain variable region CDR1, the light chain variable region CDR2, and the light chain variable region CDR3 as set forth in SEQ ID NOs: 4, 5, and 6, respectively; 9) the heavy chain variable region CDR1, the heavy chain variable region CDR2, and the heavy chain variable region CDR3 as set forth in SEQ ID NOs: 1, 2, and 82, respectively, and the light chain variable region CDR1, the light chain variable region CDR2, and the light chain variable region CDR3 as set forth in SEQ ID NOs: 4, 5, and 6, respectively; 10) the heavy chain variable region CDR1, the heavy chain variable region CDR2, and the heavy chain variable region CDR3 as set forth in SEQ ID NOs: 1, 2, and 83, respectively, and the light chain variable region CDR1, the light chain variable region CDR2, and the light chain variable region CDR3 as set forth in SEQ ID NOs: 4, 5, and 6, respectively; 11) the heavy chain variable region CDR1, the heavy chain variable region CDR2, and the heavy chain variable region CDR3 as set forth in SEQ ID NOs: 1, 2, and 3, respectively, and the light chain variable region CDR1, the light chain variable region CDR2, and the light chain variable region CDR3 as set forth in SEQ ID NOs: 4, 5, and 6, respectively; 12) the heavy chain variable region CDR1, the heavy chain variable region CDR2, and the heavy chain variable region CDR3 as set forth in SEQ ID NOs: 1, 2, and 7, respectively, and the light chain variable region CDR1, the light chain variable region CDR2, and the light chain variable region CDR3 as set forth in SEQ ID NOs: 4, 5, and 6, respectively; 13) the heavy chain variable region CDR1, the heavy chain variable region CDR2, and the heavy chain variable region CDR3 as set forth in SEQ ID NOs: 1, 2, and 8, respectively, and the light chain variable region CDR1, the light chain variable region CDR2, and the light chain variable region CDR3 as set forth in SEQ ID NOs: 4, 5, and 87, respectively; 14) the heavy chain variable region CDR1, the heavy chain variable region CDR2, and the heavy chain variable region CDR3 as set forth in SEQ ID NOs: 1, 2, and 8, respectively, and the light chain variable region CDR1, the light chain variable region CDR2, and the light chain variable region CDR3 as set forth in SEQ ID NOs: 4, 5, and 88, respectively; 15) the heavy chain variable region CDR1, the heavy chain variable region CDR2, and the heavy chain variable region CDR3 as set forth in SEQ ID NOs: 1, 2, and 8, respectively, and the light chain variable region CDR1, the light chain variable region CDR2, and the light chain variable region CDR3 as set forth in SEQ ID NOs: 4, 5, and 9, respectively; 16) the heavy chain variable region CDR1, the heavy chain variable region CDR2, and the heavy chain variable region CDR3 as set forth in SEQ ID NOs: 1, 2, and 84, respectively, and the light chain variable region CDR1, the light chain variable region CDR2, and the light chain variable region CDR3 as set forth in SEQ ID NOs: 4, 5, and 89, respectively; 17) the heavy chain variable region CDR1, the heavy chain variable region CDR2, and the heavy chain variable region CDR3 as set forth in SEQ ID NOs: 1, 2, and 85, respectively, and the light chain variable region CDR1, the light chain variable region CDR2, and the light chain variable region CDR3 as set forth in SEQ ID NOs: 4, 5, and 90, respectively; 18) the heavy chain variable region CDR1, the heavy chain variable region CDR2, and the heavy chain variable region CDR3 as set forth in SEQ ID NOs: 1, 2, and 86, respectively, and the light chain variable region CDR1, the light chain variable region CDR2, and the light chain variable region CDR3 as set forth in SEQ ID NOs: 4, 5, and 92, respectively; or 19) the heavy chain variable region CDR1, the heavy chain variable region CDR2, and the heavy chain variable region CDR3 as set forth in SEQ ID NOs: 1, 2 and 8, respectively, and the light chain variable region CDR1, the light chain variable region CDR2, and the light chain variable region CDR3 as set forth in SEQ ID NOs: 4, 5 and 91, respectively.

It should be noted that the antigen binding fragment will be composed of a partial sequence of the heavy chain variable region or light chain variable region of the antibody from which it is derived, or contain a partial sequence of the light or heavy chain variable region, and the partial sequence is sufficient to retain the same binding specificity and sufficient affinity as the antibody from which it is derived.

According to an embodiment of the present disclosure, the antibody or antigen binding fragment thereof includes a heavy chain framework region and/or a light chain framework region.

According to an embodiment of the present disclosure, at least a portion of the heavy chain framework region and/or the light chain framework region is from at least one of a murine antibody, a human antibody, a primate antibody, a bovine antibody, an equine antibody, a dairy bovine antibody, a porcine antibody, an ovine antibody, a caprine antibody, a canine antibody, a feline antibody, a rabbit-derived antibody, a camelid antibody, donkey-derived antibody, a deer-derived antibody, a mink-derived antibody, a chicken-derived antibody, a duck-derived antibody, a goose-derived antibody, a turkey-derived antibody, gamecock-derived antibody, or a mutant thereof, and preferably, at least one of a murine antibody, a human antibody, or a primate antibody.

It should be noted that, in order to further improve the biological acceptability of the antibody, the antibody may be humanized, that is, the antibody is a chimeric antibody or a humanized antibody.

As above described, one or more amino acid residues in the heavy or light chain variable region of the antibody or antigen binding fragment thereof of the present disclosure can be replaced with other amino acid residues from the same side chain family, and the retained function of the modified antibodies can be tested using the functional assay method described herein.

According to an embodiment of the present disclosure, the antibody or antigen binding fragment thereof includes: a heavy chain variable region having an amino acid sequence as set forth in one of SEQ ID NOs: 10, 12 to 13, 15 to 24, 27, and 31, or a conservative modification form thereof; and/or a light chain variable region having an amino acid sequence as set forth in one of SEQ ID NOs: 11, 14, 25 to 26, 28, and 30, or a conservative modification form thereof.

It should be noted that, without substantially affecting the CD3 binding activity of the antibody or antigen binding fragment thereof (retaining at least 95% of the activity), those skilled in the art may substitute, add, and/or delete one or more (e.g., 1, 2, 3, 4, 5, 6, 7, 8, 9, or 10 or more) amino acids in the amino acid sequence of the heavy chain or light chain variable region of the antibody or antigen binding fragment thereof of the present disclosure, to obtain sequence variants of the antibody or antigen binding fragment thereof, which are all considered to be included in the protection scope of the present disclosure. For example, amino acids with similar properties in the heavy chain variable region or light chain variable region are substituted. The above sequence variants of the present disclosure may be at least 90%, 91%, 92%, 93%, 94%, 95%, 96%, 97%, 98%, or 99% identical (or homologous) to the reference sequence.

It should be noted that the sequence identity described in the present disclosure can be measured using sequence analysis software. For example, the computer program with default parameters BLAST, in particular BLASTP or TBLASTN is used.

According to an embodiment of the present disclosure, the heavy chain variable region has an amino acid sequence with at least 90% sequence identity to an amino acid sequence as set forth in one of SEQ ID NOs: 10, 12 to 13, 15 to 24, 27, and 31; and/or the light chain variable region has an amino acid sequence with at least 90% sequence identity to an amino acid sequence as set forth in one of SEQ ID NOs: 11, 14, 25 to 26, 28, and 30.

According to an embodiment of the present disclosure, the antibody or antigen binding fragment thereof includes an amino acid sequence as set forth in a combination of the heavy chain variable region and the light chain variable region in any one of the following groups, or an amino acid sequence having at least 90% sequence identity thereto:

| Group number of the antibody or antigen binding fragment | Heavy chain variable region | Light chain variable region |
|---|---|---|
| 1 | SEQ ID NO: 15 | SEQ ID NO: 11 |
| 2 | SEQ ID NO: 16 | SEQ ID NO: 11 |
| 3 | SEQ ID NO: 17 | SEQ ID NO: 11 |
| 4 | SEQ ID NO: 17 | SEQ ID NO: 11 |
| 5 | SEQ ID NO: 19 | SEQ ID NO: 11 |
| 6 | SEQ ID NO: 20 | SEQ ID NO: 11 |
| 7 | SEQ ID NO: 21 | SEQ ID NO: 11 |
| 8 | SEQ ID NO: 22 | SEQ ID NO: 11 |
| 9 | SEQ ID NO: 23 | SEQ ID NO: 11 |
| 10 | SEQ ID NO: 24 | SEQ ID NO: 11 |
| 11 | SEQ ID NO: 10 | SEQ ID NO: 11 |
| 12 | SEQ ID NO: 12 | SEQ ID NO: 11 |
| 13 | SEQ ID NO: 13 | SEQ ID NO: 25 |
| 14 | SEQ ID NO: 13 | SEQ ID NO: 26 |
| 15 | SEQ ID NO: 13 | SEQ ID NO: 14 |
| 16 | SEQ ID NO: 27 | SEQ ID NO: 28 |
| 17 | SEQ ID NO: 29 | SEQ ID NO: 28 |
| 18 | SEQ ID NO: 13 | SEQ ID NO: 30 |
| 19 | SEQ ID NO: 31 | SEQ ID NO: 26 |

According to an embodiment of the present disclosure, the antibody or antigen binding fragment thereof further includes a constant region. The constant region including at least one of a heavy chain constant region or a light chain constant region.

According to an embodiment of the present disclosure, at least a portion of at least one of the heavy chain constant region or the light chain constant region is from at least one of a murine antibody, human antibody, primate antibody, bovine antibody, equine antibody, dairy bovine antibody, porcine antibody, ovine antibody, caprine antibody, canine antibody, feline antibody, rabbit-derived antibody, camelid antibody, donkey-derived antibody, deer-derived antibody, mink-derived antibody, chicken-derived antibody, duck-derived antibody, goose-derived antibody, turkey-derived antibody, gamecock-derived antibody, or a mutant thereof.

It should be noted that the immunoglobulins described herein can be immunoglobulin molecules of any type (e.g., IgG, IgE, IgM, IgD, and IgA), class (e.g., IgG1, IgG2, IgG3, IgG4, IgA1, and IgA2), or subclass, including engineered subclasses with altered Fc portions that provide reduced or enhanced effector cell activity. The immunoglobulins may be derived from any species.

According to an embodiment of the present disclosure, the heavy chain constant region includes a heavy chain constant region selected from IgG1, IgG2, IgG3, IgG4, IgA, IgM, IgE, or IgD; and/or the light chain constant region includes a light chain constant region selected from a κ-type light chain constant region or a λ-type light chain constant region.

In some specific embodiments, the heavy chain constant region includes a heavy chain constant region selected from human IgG, IgA, IgM, IgE, or IgD, such as human IgG1, human IgG2, human IgG3, human IgG4, human IgA, human IgM, human IgE, or human IgD.

In some specific embodiments, the heavy chain constant region includes a heavy chain constant region selected from murine IgG, IgA, IgM, IgE, or IgD, such as murine IgG1, murine IgG2a, murine IgG2b, murine IgG2c, murine IgG3, murine IgA, murine IgM, murine IgE, or murine IgD.

According to an embodiment of the present disclosure, the light chain constant region and the heavy chain constant region are both derived from a murine antibody or a mutant thereof, or a human antibody or a mutant thereof.

It should be noted that the amino acid sequences involved in the present disclosure are all shown from the N-terminus to the C-terminus.

According to an embodiment of the present disclosure, the N-terminus of the heavy chain constant region is linked to the C-terminus of the heavy chain variable region; and/or the N-terminus of the light chain constant region is linked to the N-terminus of the light chain variable region.

As described above, without substantially affecting the CD3 binding activity of the antibody or antigen binding fragment thereof (retaining at least 95% of the activity), those skilled in the art can substitute, add, and/or delete one or more (e.g., 1, 2, 3, 4, 5, 6, 7, 8, 9, 10 or more) amino acids in the amino acid sequence of the heavy chain constant region or light chain constant region of the antibody or antigen binding fragment thereof of the present disclosure, to obtain sequence variants of the antibody or antigen binding fragment thereof, which are all considered to be included in the protection scope of the present disclosure. For example, amino acids with similar properties in the heavy chain constant region or light chain constant region are substituted. The sequence variants of the present disclosure may be at least 80%, 85%, 90%, 95%, 96%, 97%, 98%, or 99% identical (or homologous) to the reference sequence.

According to an embodiment of the present disclosure, the heavy chain constant region has an amino acid sequence as set forth in SEQ ID NO: 93 or an amino acid sequence having at least 80% identity thereto; and/or the light chain constant region has an amino acid sequence as set forth in SEQ ID NO: 94 or an amino acid sequence having at least 80% identity thereto.

According to an embodiment of the present disclosure, the heavy chain constant region and the light chain constant region have amino acid sequences as set forth in SEQ ID NO: 93 and SEQ ID NO: 94, respectively.

According to an embodiment of the present disclosure, the C-terminus of the heavy chain variable region is linked to the N-terminus of the light chain variable region, or the N-terminus of the heavy chain variable region is linked to the C-terminus of the light chain variable region.

According to an embodiment of the present disclosure, the antibody or antigen binding fragment thereof further includes a first linking peptide. The C-terminus of the heavy chain variable region is linked to the N-terminus of the first linking peptide, and the C-terminus of the first linking peptide is linked to the N-terminus of the light chain variable region, or the C-terminus of the light chain variable region is linked to the N-terminus of the first linking peptide, and the C-terminus of the first linking peptide is linked to the N-terminus of the heavy chain variable region.

In some specific embodiments, the first linking peptide has an amino acid sequence as set forth in (GGGGS)n, wherein n is an integer greater than or equal to 1, and preferably n is 1, 2, 3, 4, 5, 6, 7, 8, 9, or 10.

As described above, the antibody of the present disclosure may be full-length (e.g., IgG1 or IgG4 antibody) or include only an antigen-binding portion (e.g., Fab, Fab', Fab'-SH, F(ab')₂, or Fv, scFv fragments), or may be modified to affect its function. The present disclosure includes anti-CD3 antibodies with modified glycosylation patterns. In some embodiments, it may be useful to perform modifications to remove undesirable glycosylation sites, or to produce antibodies lacking fucose moieties on the oligosaccharide chains, for example, to enhance antibody-dependent cellular cytotoxicity (ADCC) function. In other embodiments, galactosylation modification can be performed to alter complement dependent cytotoxicity (CDC).

According to an embodiment of the present disclosure, the antibody includes at least one of a full-length monoclonal antibody, chimeric antibody, humanized antibody, Fv, scFv, Fab, Fab', Fab'-SH, or F(ab')₂; and the antigen binding fragment of the antibody includes at least one of a F(ab')₂ fragment, Fab' fragment, Fab fragment, F(ab)₂ fragment, Fv fragment, scFv fragment, scFv-Fc fusion protein, scFv-Fv fusion protein, or a minimum recognition unit.

As described above, without substantially affecting the CD3 binding activity of the antibody or antigen binding fragment thereof (retaining at least 95% of the activity), those skilled in the art can substitute, add, and/or delete one or more (e.g., 1, 2, 3, 4, 5, 6, 7, 8, 9, 10 or more) amino acids in the amino acid sequence of the heavy chain or light chain, scFv fragment, Fab fragment, or scFab fragment of the antibody or antigen binding fragment thereof of the present disclosure, to obtain sequence variants of the antibody or antigen binding fragment thereof, which are all considered to be included in the protection scope of the present disclosure. For example, amino acids with similar properties in the heavy chain or light chain are substituted. The sequence variants of the present disclosure may be at least 80%, 85%, 90%, 95%, 96%, 97%, 98%, or 99% identical (or homologous) to the reference sequence.

According to an embodiment of the present disclosure, the antibody or antigen binding fragment includes: a heavy chain having an amino acid sequence as set forth in one of SEQ ID NOs: 32, 34, 35, and 37 to 46, or an amino acid sequence having at least 80% identity thereto; and/or a light chain having an amino acid sequence as set forth in one of SEQ ID NOs: 33, 36, and 47 to 48, or an amino acid sequence having at least 80% identity thereto.

According to an embodiment of the present disclosure, the antibody or antigen binding fragment thereof has an amino acid sequence as set forth in a combination of a heavy chain and a light chain in any one of the following groups, or an amino acid sequence having at least 80% sequence identity thereto:

| Group number of the antibody or antigen binding fragment thereof | Heavy chain | Light chain |
|---|---|---|
| 1 | SEQ ID NO: 37 | SEQ ID NO: 33 |
| 2 | SEQ ID NO: 38 | SEQ ID NO: 33 |
| 3 | SEQ ID NO: 39 | SEQ ID NO: 33 |
| 4 | SEQ ID NO: 40 | SEQ ID NO: 33 |
| 5 | SEQ ID NO: 41 | SEQ ID NO: 33 |
| 6 | SEQ ID NO: 42 | SEQ ID NO: 33 |
| 7 | SEQ ID NO: 43 | SEQ ID NO: 33 |
| 8 | SEQ ID NO: 44 | SEQ ID NO: 33 |
| 9 | SEQ ID NO: 45 | SEQ ID NO: 33 |
| 10 | SEQ ID NO: 46 | SEQ ID NO: 33 |
| 11 | SEQ ID NO: 32 | SEQ ID NO: 33 |
| 12 | SEQ ID NO: 34 | SEQ ID NO: 33 |
| 13 | SEQ ID NO: 35 | SEQ ID NO: 47 |
| 14 | SEQ ID NO: 35 | SEQ ID NO: 48 |
| 15 | SEQ ID NO: 35 | SEQ ID NO: 36 |
| 16 | SEQ ID NO: 95 | SEQ ID NO: 119 |
| 17 | SEQ ID NO: 96 | SEQ ID NO: 120 |
| 18 | SEQ ID NO: 97 | SEQ ID NO: 121 |
| 19 | SEQ ID NO: 98 | SEQ ID NO: 122 |

.

According to an embodiment of the present disclosure, the antibody or antigen binding fragment thereof has an amino acid sequence as set forth in any one of SEQ ID NOs: 99 to 117, or an amino acid sequence having at least 80% sequence identity thereto.

According to an embodiment of the present disclosure, the antibody or antigen binding fragment thereof has an amino acid sequence as set forth in a combination of Fab VL-CL and Fab VH-CH1 in any one of the following groups , or an amino acid sequence having at least 80% sequence identity thereto:

| Group number of antibody or antigen binding fragment | Fab VH-CH1 | Fab VL-CL |
|---|---|---|
| 1 | SEQ ID NO: 123 | SEQ ID NO: 33 |
| 2 | SEQ ID NO: 124 | SEQ ID NO: 33 |
| 3 | SEQ ID NO: 125 | SEQ ID NO: 33 |
| 4 | SEQ ID NO: 126 | SEQ ID NO: 33 |
| 5 | SEQ ID NO: 127 | SEQ ID NO: 33 |
| 6 | SEQ ID NO: 128 | SEQ ID NO: 33 |
| 7 | SEQ ID NO: 129 | SEQ ID NO: 33 |
| 8 | SEQ ID NO: 130 | SEQ ID NO: 33 |
| 9 | SEQ ID NO: 131 | SEQ ID NO: 33 |
| 10 | SEQ ID NO: 132 | SEQ ID NO: 33 |
| 11 | SEQ ID NO: 133 | SEQ ID NO: 33 |
| 12 | SEQ ID NO: 134 | SEQ ID NO: 33 |
| 13 | SEQ ID NO: 135 | SEQ ID NO: 47 |
| 14 | SEQ ID NO: 136 | SEQ ID NO: 36 |
| 15 | SEQ ID NO: 137 | SEQ ID NO: 48 |
| 16 | SEQ ID NO: 138 | SEQ ID NO: 119 |
| 17 | SEQ ID NO: 139 | SEQ ID NO: 120 |
| 18 | SEQ ID NO: 140 | SEQ ID NO: 121 |
| 19 | SEQ ID NO: 141 | SEQ ID NO: 122 |

.

According to an embodiment of the present disclosure, the antibody or antigen binding fragment thereof has an amino acid sequence as set forth in any one of SEQ ID NOs: 142 to 160, or an amino acid sequence having at least 80% sequence identity thereto.

### Multi-specific antibody

The present disclosure provides a multi-specific antibody. The multi-specific antibody includes: a first antigen binding region and a second antigen binding region. The first antigen binding region includes the above-described anti-CD3 antibody or antigen binding fragment thereof; and the second antigen binding region has a biomolecule binding activity, the biomolecule not being CD3.

As described above, the multi-specific antibodies (such as bispecific antibodies) developed based on the anti-CD3 antibody or antigen binding fragment thereof according to the first aspect of the present disclosure can be preferentially accumulated at the tumor site after being prepared into medicaments, increasing the local drug concentration at the tumor site and reducing the peripheral drug concentration, thereby reducing the risk of off-target toxicity. At the same time, the production of pro-inflammatory cytokines can be reduced and the risk of on-target toxicity of the multi-specific antibodies (such as bispecific antibodies) can be reduced. Therefore, the multi-specific antibody based on the anti-CD3 antibody or antigen binding fragment thereof of the first aspect of the present disclosure has better safety and high value in clinical application and drug development.

According to an embodiment of the present disclosure, the multi-specific antibody is selected from one of a bispecific antibody, a trispecific antibody, or a tetraspecific antibody.

The bispecific antibody is preferred.

According to an embodiment of the present disclosure, the bispecific antibody includes a symmetric bispecific antibody or an asymmetric bispecific antibody.

According to an embodiment of the present disclosure, the second antigen binding region is a binding protein of the biomolecule or a fragment thereof.

According to an embodiment of the present disclosure, the biomolecule is selected from at least one of the following: PDL1, CD47, TIGIT, CD73, CD33, CEACAM1, CEACAM5, CEACAM6, STING, WNT, Beta catenin, B7H3, VISITA, CD19, BCMA, CD22, CD20, CD123, CD38, CEA, CD25, CD46, CD138, PSCA, PSMA, PSA, MUC1, MUC16, NY-ESO-1, GD2, WT1, Mesothelin, MAGE-A3, GPC3, PRAME, Globo H, AFP, Trop2, FOLR1, SP, Sca-1, CD133, or EPCAM.

According to an embodiment of the present disclosure, the binding protein or fragment thereof is selected from at least one of an antibody or antigen binding fragment thereof, a receptor, or a ligand.

According to an embodiment of the present disclosure, the bispecific antibody is an asymmetric bispecific antibody.

According to an embodiment of the present disclosure, the first antigen binding region includes a first scFv fragment, first Fv fragment, first Fab fragment, or first Fab-Linker fragment.

According to an embodiment of the present disclosure, the first antigen binding region further includes a first Fc peptide segment.

According to an embodiment of the present disclosure, the N-terminus of the first Fc peptide segment is linked to the C-terminus of the scFv fragment or the C-terminus of the Fv fragment; or the N-terminus of the first Fc peptide segment is linked to the C-terminus of CH1 of the Fab fragment or the C-terminus of the Fab-Linker fragment.

According to an embodiment of the present disclosure, the first antigen binding region further includes a second linking peptide. The N-terminus of the first Fc peptide segment is linked to the C-terminus of the second linking peptide, and the N-terminus of the second linking peptide is linked to the C-terminus of the scFv fragment or the C-terminus of the Fv fragment; or the N-terminus of the first Fc peptide segment is linked to the C-terminus of the second linking peptide, and the N-terminus of the second linking peptide is linked to the C-terminus of CH1 of the Fab fragment or the C-terminus of Fab-Linker fragment.

In some specific embodiments, the second linking peptide has an amino acid sequence as set forth in (GGGGS)n, wherein n is an integer greater than or equal to 1, and preferably n is 1, 2, 3, 4, 5, 6, 7, 8, 9, or 10.

According to an embodiment of the present disclosure, the first Fc peptide segment is selected from a human Fc peptide segment, and preferably a human IgG1 Fc peptide.

According to an embodiment of the present disclosure, the first Fc peptide segment has an amino acid sequence as set forth in SEQ ID NO: 118; or the first Fc peptide segment has an amino acid sequence as set forth in SEQ ID NO: 166.

According to an embodiment of the present disclosure, the first scFv fragment has an amino acid sequence as set forth in any one of SEQ ID NOs: 95 to 117.

According to an embodiment of the present disclosure, the first Fab fragment has an amino acid sequence as set forth in any one of the following groups of combinations of Fab VL-CL and Fab VH-CH1:

| Group number of the first Fab fragment | Fab VH-CH1 | Fab VL-CL |
|---|---|---|
| 1 | SEQ ID NO: 123 | SEQ ID NO: 33 |
| 2 | SEQ ID NO: 124 | SEQ ID NO: 33 |
| 3 | SEQ ID NO: 125 | SEQ ID NO: 33 |
| 4 | SEQ ID NO: 126 | SEQ ID NO: 33 |
| 5 | SEQ ID NO: 127 | SEQ ID NO: 33 |
| 6 | SEQ ID NO: 128 | SEQ ID NO: 33 |
| 7 | SEQ ID NO: 129 | SEQ ID NO: 33 |
| 8 | SEQ ID NO: 130 | SEQ ID NO: 33 |
| 9 | SEQ ID NO: 131 | SEQ ID NO: 33 |
| 10 | SEQ ID NO: 132 | SEQ ID NO: 33 |
| 11 | SEQ ID NO: 133 | SEQ ID NO: 33 |
| 12 | SEQ ID NO: 134 | SEQ ID NO: 33 |
| 13 | SEQ ID NO: 135 | SEQ ID NO: 47 |
| 14 | SEQ ID NO: 136 | SEQ ID NO: 36 |
| 15 | SEQ ID NO: 137 | SEQ ID NO: 48 |
| 16 | SEQ ID NO: 138 | SEQ ID NO: 119 |
| 17 | SEQ ID NO: 139 | SEQ ID NO: 120 |
| 18 | SEQ ID NO: 140 | SEQ ID NO: 121 |
| 19 | SEQ ID NO: 141 | SEQ ID NO: 122 |

.

According to an embodiment of the present disclosure, the first Fab-Linker has an amino acid sequence as set forth in any one of SEQ ID NOs: 142 to 160.

According to an embodiment of the present disclosure, the first antigen binding region has an amino acid sequence as set forth in any one of SEQ ID NOs: 49 to 52, and 54 to 68.

According to an embodiment of the present disclosure, the biomolecule is Trop2 or PDL1.

According to an embodiment of the present disclosure, the second antigen binding region includes a first anti-Trop2 antibody or antigen binding fragment thereof; or a first anti-PDL1 antibody or an antigen binding fragment thereof.

According to an embodiment of the present disclosure, the first anti-Trop2 antibody or antigen binding fragment thereof is a second scFab fragment or a second Fab fragment of anti-Trop2; or the first anti-PDL1 antibody or antigen binding fragment thereof is a third scFab fragment or a third Fab fragment of anti-PDL1.

According to an embodiment of the present disclosure, the second scFab fragment has an amino acid sequence as set forth in SEQ ID NO: 161; or the second Fab fragment has an amino acid sequence as set forth in SEQ ID NO: 162 and 70; or the third scFab fragment has an amino acid sequence as set forth in SEQ ID NO: 163; or the third Fab fragment has an amino acid sequence as set forth in SEQ ID NO: 164 and 72.

According to an embodiment of the present disclosure, the second antigen binding region further includes a second Fc fragment. The N-terminus of the second Fc fragment is linked to the C-terminus of CH1 of the second scFab fragment, the second Fab fragment, the third scFab fragment, or the third Fab fragment.

According to an embodiment of the present disclosure, the second Fc peptide segment is selected from a human Fc peptide segment, and preferably a human IgG1 Fc peptide segment.

According to an embodiment of the present disclosure, the second Fc peptide segment has an amino acid sequence as set forth in SEQ ID NO: 165.

According to an embodiment of the present disclosure, the first Fc peptide segment and the second Fc peptide segment are linked via a knob-into-hole structure. According to an embodiment of the present disclosure, the second antigen binding region has an amino acid sequence as set forth in SEQ ID NO: 161 or 163; or the second antigen binding region has an amino acid sequence as set forth in SEQ ID NO: 162 and 70; or the second antigen binding region has an amino acid sequence as set forth in SEQ ID NO: 164 and 72.

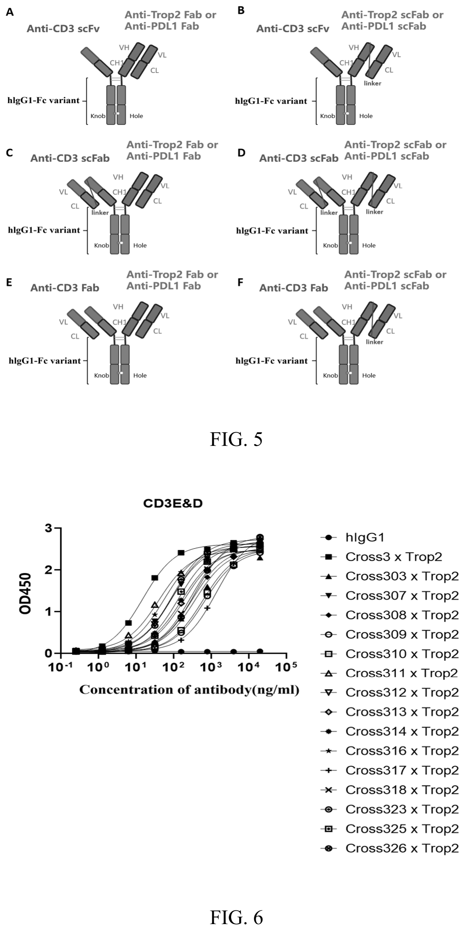

In some optional embodiments of the present disclosure, the multi-specific antibody of the present disclosure has the structure shown in FIG. 5.

In some optional embodiments of the present disclosure, as shown in FIG. 5A, the multi-specific antibody of the present disclosure includes: a first peptide chain having an amino acid sequence as set forth in any one of SEQ ID NOs: 49 to 52 and 54 to 68, a second peptide chain having an amino acid sequence as set forth in SEQ ID NO: 69, and a third peptide chain having an amino acid sequence as set forth in SEQ ID NO: 70; or a second peptide chain having an amino acid sequence as set forth in SEQ ID NO: 71, and a third peptide chain having an amino acid sequence as set forth in SEQ ID NO: 72.

In some optional embodiments of the present disclosure, as shown in FIG. 5B, the multi-specific antibody of the present disclosure includes: a first peptide chain having an amino acid sequence as set forth in any one of SEQ ID NOs: 49 to 52 and 54 to 68, and a second peptide chain having an amino acid sequence as set forth in SEQ ID NO: 161 or 163.

In some optional embodiments of the present disclosure, as shown in FIG. 5C, the multi-specific antibody of the present disclosure includes: a first peptide chain having an amino acid sequence as set forth in any one of SEQ ID NOs: 142 to 160, a second peptide chain having an amino acid sequence as set forth in SEQ ID NO: 69, and a third peptide chain having an amino acid sequence as set forth in SEQ ID NO: 70; or a second peptide chain having an amino acid sequence as set forth in SEQ ID NO: 71, and a third peptide chain having an amino acid sequence as set forth in SEQ ID NO: 72.

In some optional embodiments of the present disclosure, as shown in FIG. 5D, the multi-specific antibody of the present disclosure includes: a first peptide chain having an amino acid sequence as set forth in any one of SEQ ID NOs: 142 to 160, and a second peptide chain having an amino acid sequence as set forth in SEQ ID NO: 161 or 163.

In some optional embodiments of the present disclosure, as shown in FIG. 5E, the multi-specific antibody of the present disclosure includes: a first peptide chain having an amino acid sequence as set forth in SEQ ID NO: 70, a third peptide chain having an amino acid sequence as set forth SEQ ID NO: 69, and a fourth peptide chain having an amino acid sequence as set forth in SEQ ID NO: 70; or a first peptide chain having an amino acid sequence as set forth in SEQ ID NO: 72, a third peptide chain having an amino acid sequence as set forth in SEQ ID NO: 71, a fourth peptide chain having an amino acid sequence as set forth in SEQ ID NO: 72; and a second peptide chain including a Fab VH-CH1 having an amino acid sequence as set forth in SEQ ID NO: 162 or 164 and a scFv having an amino acid sequence as set forth in SEQ ID NOs: 99 to 117, the C-terminus of the Fab VH-CH1 being linked to the N-terminus of the scFv.

### Conjugate

The present disclosure provides a conjugate. The conjugate includes: the above-described antibody or antigen binding fragment thereof or the above-described multi-specific antibody; and a conjugate moiety, the conjugated moiety being linked to the antibody or antigen binding fragment thereof or the multi-specific antibody.

As described above, the anti-CD3 antibodies or antigen binding fragments thereof of the embodiments of the present disclosure can be used to prepare multi-specific antibody drug, such as bispecific antibody drug. On the one hand, the prepared bispecific antibody drug can be preferentially accumulated at the tumor site, increasing the local drug concentration at the tumor site and reducing the peripheral drug concentration, thereby reducing the risk of off-target toxicity. On the other hand, the bispecific antibody can reduce the production of pro-inflammatory cytokines, reducing the risk of on-target toxicity of the bispecific antibody. Furthermore, the drugs prepared using the anti-CD3 antibodies or antigen binding fragments thereof can be used to prevent and/or a treat CD3-related disease. The CD3 bispecific antibody drugs developed based on the anti-CD3 antibodies or antigen binding fragments thereof are safer and can be used to prevent and/or treat a cancer or tumor or infection or autoimmune disease. Therefore, the conjugate including the anti-CD3 antibody or antigen binding fragment thereof of the first aspect of the present disclosure and the multi-specific antibody of the second aspect of the present disclosure has good clinical application value and drug development value.

According to an embodiment of the present disclosure, the conjugated moiety includes at least one selected from a carrier, a medicament, a toxin, a cytokine, a protein tag, a modifier, a therapeutic agent, or a chemotherapeutic agent.

### Nucleic acid

The present disclosure provides a nucleic acid. The nucleic acid encodes the above-described antibody or antigen binding fragment thereof or the above-described multi-specific antibody.

According to an embodiment of the present disclosure, the antibody or antigen binding fragment thereof of the first aspect of the present disclosure or the multi-specific antibody of the second aspect of the present disclosure encoded by the nucleic acid is suitable for developing a safer monoclonal antibody or bispecific antibody drug. Furthermore, the protein encoded by the nucleic acid can be used to prevent and/or treat a CD3-related disease or prevent and/or treat a cancer or tumor or infection or autoimmune disease.

It should be noted that, for the nucleic acid molecules described herein, those skilled in the art should understand that they actually include any one or both of the complementary double strands. For convenience, although only one chain is shown in most cases herein, the other chain complementary thereto is actually also disclosed. In addition, the molecular sequences in the present disclosure include DNA forms or RNA forms, and disclosure of one of them means that the other is also disclosed.

### Vector or transformant

The present disclosure provides a vector or transformant. The vector or transformant contains the above-described nucleic acid. The vector or transformant may include optional control sequences operably linked to the nucleic acid molecule. The control sequence is one or more control sequences that can direct the expression of the nucleic acid molecule in the host. The vector or transformant thus constructed can effectively express the antibody or antigen binding fragment thereof of the first aspect of the present disclosure or the multi-specific antibody of the second aspect of the present disclosure, and can obtain the conjugate of the third aspect of the present disclosure.

When the above-described nucleic acid molecule is linked to the vector or transformant, such as an expression vector, the nucleic acid molecule can be directly or indirectly linked to the control elements on the expression vector, as long as these control elements can control the translation and expression of the nucleic acid molecule. Of course, these control elements may be derived directly from the vector itself, or they can be exogenous, that is, not from the vector itself. The nucleic acid molecule is operably linked to the control element.

According to an embodiment of the present disclosure, the vector may refer to a cloning vector or an expression vector, which may be obtained by operably linking the nucleic acid to a commercially available vector (such as a plasmid or a viral vector). The vector in the present disclosure is not particularly limited, and commonly used plasmids can be used, such as pSeTag2, PEE14, pMH3, etc.

As used herein, the term "operably linked" means that the exogenous gene is linked to the vector. In this way, the control elements in the vector, such as transcription control sequences and translation control sequences, can play their intended function of regulating the transcription and translation of the exogenous gene. Commonly used vectors include viral vectors, plasmids, phages, and the like. After the expression vector according to some specific embodiments of the present disclosure is introduced into appropriate recipient cells, the expression of the above-described nucleic acid molecules can be effectively achieved under the mediation of the regulatory system, thereby achieving large-scale *in vitro* production of the protein encoded by the nucleic acid molecules.

According to an embodiment of the present disclosure, the vector is a eukaryotic carrier or a prokaryotic carrier.

According to an embodiment of the present disclosure, the vector includes at least one selected from a plasmid vector, an adenovirus vector, a lentivirus vector, or an adeno-associated virus vector.

### Cell

The present disclosure provides a cell. The cell carries the above-described nucleic acid, or the above-described vector or transformant; or expresses the above-described antibody or antigen binding fragment thereof or the above-described multi-specific antibody. Thus, according to an embodiment of the present disclosure, the cells can efficiently express the antibody or antigen binding fragment thereof of the first aspect of the present disclosure or the multi-specific antibody of the second aspect of the present disclosure under suitable conditions. Furthermore, an antibody or antigen binding fragment thereof or multi-specific antibody that can bind to human CD3 protein and has improved safety can be obtained.

According to an embodiment of the present disclosure, the cell is a prokaryotic cell, a eukaryotic cell, or a bacteriophage.

According to an embodiment of the present disclosure, the prokaryotic cell is *Escherichia coli, Bacillus subtilis, Streptomyces,* or *Proteus mirabilis.*

According to an embodiment of the present disclosure, the eukaryotic cell is a fungus, an insect cell, a plant cell, or a mammalian cell.

According to an embodiment of the present disclosure, the fungus is *Pichia pastoris, Saccharomyces cerevisiae, Schizosaccharomyces pombe,* or *Trichoderma.*

According to an embodiment of the present disclosure, the insect cell is a *Spodoptera frugiperda* cell. According to an embodiment of the present disclosure, the plant cell is a tobacco plant cell. According to an embodiment of the present disclosure, the mammalian cell is a BHK cell, a CHO cell, a COS cell, a myeloma cell, or a human embryonic kidney 293 cell; and does not include animal germ cells, fertilized eggs, or embryonic stem cells.

According to an embodiment of the present disclosure, the cell is a mammalian cell.

According to an embodiment of the present disclosure, the cell is a BHK cell, a CHO cell, a COS cell, or a NSO cell.

It should be noted that the "suitable conditions" described in the specification of the present disclosure refer to conditions suitable for the expression of the antibody or antigen binding fragment thereof or multi-specific antibody of the present disclosure. It is readily understood by those skilled in the art that conditions suitable for the expression of antibodies or antigen binding fragments thereof or multi-specific antibodies include, but are not limited to, suitable transformation or transfection methods, suitable transformation or transfection conditions, healthy host cell status, suitable host cell density, suitable cell culture environment, and suitable cell culture time. The "suitable conditions" are not particularly limited, and those skilled in the art can optimize the most suitable conditions for expressing the recombinant antibody according to the specific environment of the laboratory.

### Pharmaceutical composition

The present disclosure provides a pharmaceutical composition. The pharmaceutical composition includes: the above-described antibody or antigen binding fragment thereof, the above-described multi-specific antibody, the above-described conjugate, the above-described nucleic acid, or the above-described vector or transformant. Thus, the obtained medicament can be further used for the prevention and/or treatment of a CD3-related disease, or further used for the prevention and/or treatment of a CD3-related disease and/or cancer or tumor or infection or autoimmune disease.

According to an embodiment of the present disclosure, the composition further includes a pharmaceutically acceptable adjuvant.

According to an embodiment of the present disclosure, the adjuvant includes: one or more pharmaceutically acceptable excipients, diluents, stabilizers, or carriers.

According to an embodiment of the present disclosure, the pharmaceutical composition is an injection.

It should be noted that the pharmaceutical composition includes combinations separated in time and/or space, as long as they can work together to achieve the purpose of the present disclosure. For example, the components contained in the composition can be administered to the subject as a whole, or separately. When the components contained in the composition are administered to the subject separately, the individual components can be administered to the subject simultaneously or sequentially.

The pharmaceutical composition of the present disclosure contains a safe and effective amount of the active ingredient of the present disclosure and pharmaceutically acceptable adjuvants. Such adjuvants include, but are not limited to, saline, buffer, dextrose, water, glycerol, ethanol, and combinations thereof. Generally, pharmaceutical preparations should be compatible with the mode of administration. The dosage forms of the pharmaceutical composition of the present disclosure are injections, oral preparations (tablets, capsules, oral liquids), transdermal preparations, and sustained-release preparations. For example, it can be prepared by conventional methods using physiological saline or an aqueous solution containing glucose and other adjuvants. The pharmaceutical composition is preferably manufactured under sterile conditions.

The effective amount of the active ingredient of the present disclosure may vary depending on the mode of administration and the severity of the disease to be treated. The selection of the preferred effective amount can be determined by those skilled in the art based on various factors (e.g., through clinical trials). The factors include, but are not limited to: pharmacokinetic parameters of the active ingredient, such as bioavailability, metabolism, half-life, etc.; severity of the disease to be treated, weight of the patient, immune status of the patient, route of administration, etc. For example, several divided doses may be administered daily or the dose may be proportionally reduced depending on the exigencies of the therapeutic situation.

The pharmaceutically acceptable adjuvants of the present disclosure include (but are not limited to): water, saline, liposomes, lipids, proteins, protein-antibody conjugates, peptides, cellulose, nanogels, or combinations thereof. The selection of the carrier should be compatible with the mode of administration, which is well known to those skilled in the art.

### Kit

The present disclosure provides a kit. The kit includes the above-described antibody or antigen binding fragment thereof or the above-described multi-specific antibody. As described above, the antibodies or antigen binding fragments thereof according to the embodiments of the present disclosure can effectively and specifically bind to CD3. The CD3-related kit developed by utilizing this property can be used in CD3-related research.

The kit can effectively detect, enrich, or separate and purify CD3 in biological samples, and is further used in scientific research, such as qualitatively or quantitatively detecting CD3 protein molecules in biological samples. More specifically, it can be used in kits for immunoblotting, immunoprecipitation, and the like that involve detection using the specific binding properties of CD3 and antibodies or binding fragments thereof. These kits may contain any one or more of the following: antagonists, CD3 antibodies, or medicament reference materials; protein purification columns; immunoglobulin affinity purification buffers; and cell assay diluents. CD3 antibodies can be used in various types of diagnostic tests, such as in vitro or in vivo detection of the presence of various diseases, or medicaments, toxins, or other proteins. For example, CD3-related diseases can be detected by testing the serum or blood of the subject.

### Use in the manufacture of a kit

The present disclosure provides use of the above-described antibody or antigen binding fragment thereof or the above-described multi-specific antibody in the manufacture of a kit for detecting CD3.

As described above, the antibodies or antigen binding fragments thereof according to the embodiments of the present disclosure can specifically bind to CD3. Therefore, the antibodies or antigen binding fragments thereof can be used to detect CD3. Furthermore, it can be used to prepare CD3-related kits and used in scientific research, such as qualitative or quantitative detection of CD3 protein molecules in biological samples. More specifically, it can be used in kits for immunoblotting, immunoprecipitation, and the like that involve detection using the specific binding properties of CD3 and antibodies or binding fragments thereof. These kits may contain any one or more of the following: antagonists, CD3 antibodies, or medicament reference materials; protein purification columns; immunoglobulin affinity purification buffers; and cell assay diluents. CD3 antibodies or antigen fragments can be used in various types of diagnostic tests, such as in vitro or in vivo detection of the presence of various diseases or medicaments, toxins or other proteins. For example, CD3-related diseases can be detected by testing the serum or blood of the subject.

### Use in the manufacture of a medicament

The present disclosure provides use of the above-described antibody or antigen binding fragment thereof, the above-described multi-specific antibody, the above-described conjugate, the above-described nucleic acid, the above-described vector or transformant, or the above-described pharmaceutical composition in the manufacture of a medicament for regulating an immune response and/or inhibiting tumor growth, or for preventing and/or treating a CD3-related disease and/or cancer or tumor or infection or autoimmune disease.

According to an embodiment of the present disclosure, the cancer or tumor or infection or autoimmune disease is a Trop2- or PDL1-related disease.

According to an embodiment of the present disclosure, the cancer or tumor is at least one of lung cancer, liver cancer, ovarian cancer, cervical cancer, skin cancer, bladder cancer, colon cancer, rectal cancer, breast cancer, pancreatic cancer, prostate cancer, glioma, renal cancer, gastric cancer, esophageal cancer, oral squamous cell carcinoma, or head-and-neck cancer

According to an embodiment of the present disclosure, the cancer or tumor is at least one of non-small cell lung cancer, malignant melanoma, renal cell carcinoma, head-and-neck squamous cell carcinoma, urothelial carcinoma, colorectal cancer, liver cancer, or classical Hodgkin's lymphoma.

### A method for treating diseases

The present disclosure provides a method for regulating an immune response and/or inhibiting tumor growth or preventing and/or treating a CD3-related disease and/or cancer, tumor, infection, or autoimmune disease.

According to an embodiment of the present disclosure, the method includes: administering to a subject a pharmaceutically acceptable amount of the above-described antibody or antigen binding fragment thereof, the above-described multi-specific antibody, the above-described conjugate, the above-described nucleic acid, the above-described vector or transformant, or the above-described pharmaceutical composition.

It should be noted that the terms "subject", "individual", and "patient" may be used interchangeably herein, and refer to a mammal being evaluated for treatment and/or being treated. In one embodiment, the mammal is a human. The terms "subject", "individual", and "patient" include, but are not limited to, individuals with cancer, individuals with autoimmune diseases, individuals with pathogen infection, and the like. The subject can be a human, but also includes other mammals, particularly mammals that can be used as laboratory models of human disease, such as mice, rats, etc.

The effective amount of the antibody or antigen binding fragment thereof, multi-specific antibody, conjugate, nucleic acid, vector or transformant, or pharmaceutical composition of the present disclosure may vary depending on the mode of administration and the severity of the disease to be treated. The selection of the preferred effective amount can be determined by those skilled in the art based on various factors (e.g., through clinical trials). The factors include, but are not limited to: pharmacokinetic parameters of the active ingredient such as bioavailability, metabolism, half-life, etc.; severity of the disease to be treated, weight of the patient, immune status of the patient, route of administration, etc. For example, several divided doses may be administered daily or the dose may be proportionally reduced depending on the exigencies of the therapeutic situation.

According to an embodiment of the present disclosure, the cancer or tumor or infection or autoimmune disease is a Trop2- or PDL1-related disease.

According to an embodiment of the present disclosure, the cancer or tumor is at least one of lung cancer, liver cancer, ovarian cancer, cervical cancer, skin cancer, bladder cancer, colon cancer, rectal cancer, breast cancer, pancreatic cancer, prostate cancer, glioma, renal cancer, gastric cancer, esophageal cancer, oral squamous cell carcinoma, or head-and-neck cancer.

According to an embodiment of the present disclosure, the cancer or tumor is at least one of non-small cell lung cancer, malignant melanoma, renal cell carcinoma, head-and-neck squamous cell carcinoma, urothelial carcinoma, colorectal cancer, liver cancer, or classical Hodgkin's lymphoma.

The description of the sequences involved in the present disclosure are detailed in Table 1.

**[Table 1] Amino acid sequence description table**

| SEQ ID NO: | Sequence | Description |
|---|---|---|
| 1 | GFTFNTYA | Cross303/307/308/309/310/311 /312/313/314/316/317/318/323/ 325/326/Cross3ZH07/Cross3Z H09/Cross3ZH16/Cross3ZH23 HCDR1 |
| 2 | IRSKYNNYAT | Cross303/307/308/309/310/311 /312/313/314/316/317/318/323/ 325/326/Cross3ZH07/Cross3Z H09/Cross3ZH16/Cross3ZH23 HCDR2 |
| 3 | VRHGNFGNSYQSWFAY | Cross313 HCDR3 |
| 4 | TGAVTTSNY | Cross303/307/308/309/310/311 /312/313/314/316/317/318/323/ 325/326/Cross3ZH07/Cross3Z H09/Cross3ZH16/Cross3ZH23 LCDR1 |
| 5 | GTN | Cross303/307/308/309/310/311 /312/313/314/316/317/318/323/ 325/326/Cross3ZH07/Cross3Z H09/Cross3ZH16/Cross3ZH23 LCDR2 |
| 6 | ALWYSNLWV | Cross303/307/308/309/310/311 /312/313/314/316/317/318 LCDR3 |
| 7 | VRHGNFGNSYVSWFLY | Cross316 HCDR3 |
| 8 | VRHGNFGNSYVSWFAY | Cross325/323/326/Cross3ZH16 HCDR3 |
| 9 | ALWYSNDWV | Cross325 LCDR3 |
| 10 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYQSWFAYWGQGTLVTVSS | Cross313 VH |
| 11 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNLWVFGGGTKL TVL | Cross3/303/307/308/309/310/3 11/312/313/314//316/317/318 VL |
| 12 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFLYWGQGTLVTVSS | Cross316 VH |
| 13 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSS | Cross3/323/325/326/ZH16 VH |
| 14 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNDWVFGGGTKL TVL | Cross325 VL |
| 15 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGMF GNSYVSWFAYWGQGTLVTVSS | Cross303/ZH07 VH |
| 16 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNEYVSWFAYWGQGTLVTVSS | Cross307 VH |
| 17 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNPYVSWFAYWGQGTLVTVSS | Cross308 VH |
| 18 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNVYVSWFAYWGQGTLVTVSS | Cross309 VH |
| 19 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNYYVSWFAYWGQGTLVTVSS | Cross310 VH |
| 20 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSEVSWFAYWGQGTLVTVSS | Cross311 VH |
| 21 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYHSWFAYWGQGTLVTVSS | Cross312 VH |
| 22 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYSSWFAYWGQGTLVTVSS | Cross314 VH |
| 23 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAGWGQGTLVTVSS | Cross317 VH |
| 24 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAMWGQGTLVTVSS | Cross318 VH |
| 25 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYPNLWVFGGGTKL TVL | Cross323 VL |
| 26 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNPWVFGGGTKL TVL | Cross326/ZH23 VL |
| 27 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGMF GNSYVSWFAYWGQGTLVTVSS | Cross3ZH07 VH |
| 28 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYPNPWVFGGGTKL TVL | Cross3ZH07/ZH09 VL |
| 29 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFLYWGQGTLVTVSS | Cross3ZH09 VH |
| 30 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYPNDWVFGGGTKL TVL | Cross3ZH16 VL |
| 31 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNDYVSWFAYWGQGTLVTVSS | Cross3ZH23 VH |
| 32 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYQSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross313 heavy chain |
| 33 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNLWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GEC | Cross3/303/307/308/309/310/3 11/312/313/314//316/317/318 light chain |
| 34 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFLYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross316 heavy chain |
| 35 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross3/323/325/326 heavy chain |
| 36 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNDWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GEC | Cross325 light chain |
| 37 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGMF GNSYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross303 heavy chain |
| 38 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNEYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross307 heavy chain |
| 39 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNPYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross308 heavy chain |
| 40 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNVYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross309 heavy chain |
| 41 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNYYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross310 heavy chain |
| 42 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSEVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross311 heavy chain |
| 43 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYHSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross312 heavy chain |
| 44 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYSSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross314 heavy chain |
| 45 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAGWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross317 heavy chain |
| 46 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAMWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross318 heavy chain |
| 47 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYPNLWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GEC | Cross323 light chain |
| 48 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNPWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GEC | Cross326 light chain |
| 49 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYQSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross313-kbss |
| 50 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFLYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross316-kbss |
| 51 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNDWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross325-kbss |
| 52 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross3-kbss |
| 53 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGMF GNSYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross303-kbss |
| 54 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNEYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross307-kbss |
| 55 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNPYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross308-kbss |
| 56 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNVYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross309-kbss |
| 57 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNYYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross310-kbss |
| 58 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSEVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross311-kbss |
| 59 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYHSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross312-kbss |
| 60 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYSSWFAYWGQGTLVTVSSGGGGSGGGGSGGGGSELVVTQEPSLTVSPGGT VTLTCRSSTGAVTTSNYANWVQQKPGQAPRGLIGGTNKRAPGTPARFSGSLLG GKAALTLSGVQPEDEAEYYCALWYSNLWVFGGGTKLTVLGGGGSPKSSDKTH TCPPCPAPEAAGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALP APIEKTISKAKGQPREPQVYTLPPCREEMTKNQVSLWCLVKGFYPSDIAVEWES NGQPENNYKTTPPVLDSDGSFFLYSKLTVDKSRWQQGNVFSCSVMHEALHNH YTQKSLSLSPGK | Cross314-kbss |
| 61 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAGWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross317-kbss |
| 62 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAMWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross318-kbss |
| 63 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYPNLWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross323-kbss |
| 64 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNPWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross326-kbss |
| 65 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGMF GNSYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYPNPWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross3ZH07-kbss |
| 66 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFLYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYPNPWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross3ZH09-kbss |
| 67 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYPNDWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross3ZH16-kbss |
| 68 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNDYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNPWVFGGGTKLTVLGGGGSPKSSDKT HTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHN HYTQKSLSLSPGK | Cross3ZH23-kbss |
| 69 | QVQLQQSGSELKKPGASVKVSCKASGYTFTNYGMNWVKQAPGQGLKWMG WINTYTGEPTYTDDFKGRFAFSLDTSVSTAYLQISSLKADDTAVYFCARGGFGS SYWYFDVWGQGSLVTVSSASTKGPSVFPLAPSSKSTSGGTAALGCLVKDYFPE PVTVSWNSGALTSGVHTFPAVLQSSGLYSLSSVVTVPSSSLGTQTYICNVNHKP SNTKVDKKVEPKSSDKTHTCPPCPAPEAAGGPSVFLFPPKPKDTLMISRTPEVT CVVVDVSHEDPEVKFNWYVDGVEVHNAKTKPREEQYNSTYRVVSVLTVLHQ DWLNGKEYKCKVSNKALPAPIEKTISKAKGQPREPQVCTLPPSREEMTKNQVS LSCAVKGFYPSDIAVEWESNGQPENNYKTTPPVLDSDGSFFLVSKLTVDKSRWQ QGNVFSCSVMHEALHNHYTQKSLSLSPGK | Trop2 antibody heavy chain |
| 70 | DIQLTQSPSSLSASVGDRVSITCKASQDVSIAVAWYQQKPGKAPKLLIYSASYRY TGVPDRFSGSGSGTDFTLTISSLQPEDFAVYYCQQHYITPLTFGAGTKVEIKRTV AAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC | Trop2 antibody light chain |
| 71 | QVQLVQSGAEVKKPGASVKVSCKASGYSFTGYYMHWVRQAPGQGLEWIGRI NPYNGATTYSQNFKDRATMTVDTSISTAYMELSRLRSDDTAVYYCVPTGMGYF LMDYWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNT KVDKKVEPKSCDKTHTCPPCPAPEAAGGPSVFLFPPKPKDTLMISRTPEVTCVV VDVSHEDPEVKFNWYVDGVEVHNAKTKPREEQYNSTYRVVSVLTVLHQDWL NGKEYKCKVSNKALPAPIEKTISKAKGQPREPQVCTLPPSREEMTKNQVSLSCA VKGFYPSDIAVEWESNGQPENNYKTTPPVLDSDGSFFLVSKLTVDKSRWQQGN VFSCSVMHEALHNHYTQKSLSLSPGK | PDL1 antibody heavy chain |
| 72 | DIQLTQSPSFLSASVGDRVTITCKASQDVGSAVVWYQQKPGKAPKLLIYWASTR HTGVPSRFSGSGSGTEFTLTISSLQPEDFATYFCQQFTSYPTFGQGTKLEIKRTVA APSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC | PDL1 antibody light chain |
| 73 | MQSGTHWRVLGLCLLSVGVWGQDGNEEMGGITQTPYKVSISGTTVILTCPQYP GSEILWQHNDKNIGGDEDDKNIGSDEDHLSLKEFSELEQSGYYVCYPRGSKPE DANFYLYLRARVCENCMEMDVMSVATIVIVDICITGGLLLLVYYWSKNRKAKA KPVTRGAGAGGRQRGQNKERPPPVPNPDYEPIRKGQRDLYSGLNQRRI | CD3e |
| 74 | VRHGMFGNSYVSWFAY | Cross303 HCDR3 |
| 75 | VRHGNFGNEYVSWFAY | Cross307 HCDR3 |
| 76 | VRHGNFGNPYVSWFAY | Cross308 HCDR3 |
| 77 | VRHGNFGNVYVSWFAY | Cross309 HCDR3 |
| 78 | VRHGNFGNYYVSWFAY | Cross310 HCDR3 |
| 79 | VRHGNFGNSEVSWFAY | Cross311 HCDR3 |
| 80 | VRHGNFGNSYHSWFAY | Cross312 HCDR3 |
| 81 | VRHGNFGNSYSSWFAY | Cross314 HCDR3 |
| 82 | VRHGNFGNSYVSWFAG | Cross317 HCDR3 |
| 83 | VRHGNFGNSYVSWFAM | Cross318 HCDR3 |
| 84 | VRHGMFGNSYVSWFAY | Cross3ZH07 HCDR3 |
| 85 | VRHGNFGNSYVSWFLY | Cross3ZH09 HCDR3 |
| 86 | VRHGNFGNDYVSWFAY | Cross3ZH23 HCDR3 |
| 87 | ALWYPNLWV | Cross323 LCDR3 |
| 88 | ALWYSNPWV | Cross326 LCDR3 |
| 89 | ALWYPNPWV | Cross3ZH07 LCDR3 |
| 90 | ALWYPNPWV | Cross3ZH09 LCDR3 |
| 91 | ALWYPNDWV | Cross3ZH16 LCDR3 |
| 92 | ALWYSNPWV | Cross3ZH23 LCDR3 |
| 93 | ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPA PIEKTISKAKGQPREPQVYTLPPSREEMTKNQVSLTCLVKGFYPSDIAVEWESN GQPENNYKTTPPVLDSDGSFFLYSKLTVDKSRWQQGNVFSCSVMHEALHNHY TQKSLSLSPGK | Heavy chain constant region of CD3 antibody |
| 94 | | Light chain constant region of CD3 antibody |
| 95 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGMF GNSYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross3ZH07 heavy chain |
| 96 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFLYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross3ZH09 heavy chain |
| 97 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross3ZH16 heavy chain |
| 98 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNDYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRW QQGNVFSCSVMHEALHNHYTQKSLSLSPGK | Cross3ZH23 heavy chain |
| 99 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGMF GNSYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVL | Cross303 scFv |
| 100 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNEYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVL | Cross307 scFv |
| 101 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNPYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVL | Cross308 scFv |
| 102 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNVYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVL | Cross309 scFv |
| 103 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNYYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVL | Cross310 scFv |
| 104 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSEVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVL | Cross311 scFv |
| 105 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYHSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVL | Cross312 scFv |
| 106 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYQSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVL | Cross313 scFv |
| 107 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYSSWFAYWGQGTLVTVSSGGGGSGGGGSGGGGSELVVTQEPSLTVSPGGT VTLTCRSSTGAVTTSNYANWVQQKPGQAPRGLIGGTNKRAPGTPARFSGSLLG GKAALTLSGVQPEDEAEYYCALWYSNLWVFGGGTKLTVL | Cross314 scFv |
| 108 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFLYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVL | Cross316 scFv |
| 109 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAGWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVL | Cross317 scFv |
| 110 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAMWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNLWVFGGGTKLTVL | Cross318 scFv |
| 111 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYPNLWVFGGGTKLTVL | Cross323 scFv |
| 112 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNDWVFGGGTKLTVL | Cross325 scFv |
| 113 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNPWVFGGGTKLTVL | Cross326 scFv |
| 114 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGMF GNSYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYPNPWVFGGGTKLTVL | Cross3ZH07 scFv |
| 115 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFLYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYPNPWVFGGGTKLTVL | Cross3ZH09 scFv |
| 116 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYPNDWVFGGGTKLTVL | Cross3ZH16 scFv |
| 117 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNDYVSWFAYWGQGTLVTVSSGGGGSGGGGSGGGGSELVVTQEPSLTVSPGG TVTLTCRSSTGAVTTSNYANWVQQKPGQAPRGLIGGTNKRAPGTPARFSGSLL GGKAALTLSGVQPEDEAEYYCALWYSNPWVFGGGTKLTVL | Cross3ZH23 scFv |
| 118 | PKSSDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKC KVSNKALPAPIEKTISKAKGQPREPQVYTLPPCREEMTKNQVSLWCLVKGFYPS DIAVEWESNGQPENNYKTTPPVLDSDGSFFLYSKLTVDKSRWQQGNVFSCSVM HEALHNHYTQKSLSLSPGK | Corresponding first Fc peptide segment when CD3 binding region is an scFv structure |
| 119 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYPNPWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GEC | Cross3ZH07 light chain |
| 120 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYPNPWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GEC | Cross3ZH09 light chain |
| 121 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYPNDWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GEC | Cross3ZH16 light chain |
| 122 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNPWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GEC | Cross3ZH23 light chain |
| 123 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGMF GNSYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross303 Fab VH-CH1 |
| 124 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNEYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross307Fab VH-CH1 |
| 125 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNPYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross308Fab VH-CH1 |
| 126 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNVYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross309Fab VH-CH1 |
| 127 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNYYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross310Fab VH-CH1 |
| 128 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSEVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross311Fab VH-CH1 |
| 129 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYHSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross312Fab VH-CH1 |
| 130 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYQSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross313Fab VH-CH1 |
| 131 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYSSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross314Fab VH-CH1 |
| 132 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFLYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross316Fab VH-CH1 |
| 133 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAGWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross317Fab VH-CH1 |
| 134 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAMWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross318Fab VH-CH1 |
| 135 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross323Fab VH-CH1 |
| 136 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross325Fab VH-CH1 |
| 137 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross326Fab VH-CH1 |
| 138 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGMF GNSYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross3ZH07Fab VH-CH1 |
| 139 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFLYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross3ZH09Fab VH-CH1 |
| 140 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNSYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross3ZH16Fab VH-CH1 |
| 141 | EVQLLESGGGLVQPGGSLKLSCAASGFTFNTYAMNWVRQAPGKGLEWVARIR SKYNNYATYYADSVKDRFTISRDDSKNTAYLQMNNLKTEDTAVYYCVRHGNF GNDYVSWFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICNVNH KPSNTKVDKKVE | Cross3ZH23Fab VH-CH1 |
| 142 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNLWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGMFGNSYVSWFAYW GQGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKV E | Cross303 scFab |
| 143 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNLWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNEYVSWFAYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE | Cross307scFab |
| 163 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNLWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNPYVSWFAYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE | Cross308scFab |
| 145 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNLWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNVYVSWFAYW GQGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKV E | Cross309scFab |
| 146 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNLWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNYYVSWFAYW GQGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKV E | Cross310scFab |
| 147 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNLWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNSEVSWFAYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE | Cross311scFab |
| 148 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNLWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNSYHSWFAYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE | Cross312scFab |
| 149 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNLWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNSYQSWFAYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE | Cross313scFab |
| 150 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNLWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNSYSSWFAYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE | Cross314scFab |
| 151 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNLWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNSYVSWFLYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE | Cross316scFab |
| 152 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNLWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNSYVSWFAGWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE | Cross317scFab |
| 153 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNLWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNSYVSWFAMW GQGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKV E | Cross318scFab |
| 154 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYPNLWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNSYVSWFAYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE | Cross323scFab |
| 155 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNDWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNSYVSWFAYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE | Cross325scFab |
| 156 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNPWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNSYVSWFAYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE | Cross326scFab |
| 157 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYPNPWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGMFGNSYVSWFAYW GQGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKV E | Cross3ZH07scFab |
| 158 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYPNPWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNSYVSWFLYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE | Cross3ZH09scFab |
| 159 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYPNDWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNSYVSWFAYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE | Cross3ZH16scFab |
| 160 | ELVVTQEPSLTVSPGGTVTLTCRSSTGAVTTSNYANWVQQKPGQAPRGLIGGTN KRAPGTPARFSGSLLGGKAALTLSGVQPEDEAEYYCALWYSNPWVFGGGTKL TVLRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GECGGGGSEGKSSGSGSESKSTEGKSSGSGSESKSTGGGGSEVQLLESGGGLV QPGGSLKLSCAASGFTFNTYAMNWVRQAPGKGLEWVARIRSKYNNYATYYAD SVKDRFTISRDDSKNTAYLQMNNLKTEDTAVYYCVRHGNFGNDYVSWFAYW GQGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKV E | Cross3ZH23scFab |
| 161 | RTVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRGEC GGGGSEGKSSGSGSESKSTEGKSSGSGSESKSTGGGGSQVQLQQSGSELKKPG ASVKVSCKASGYTFTNYGMNWVKQAPGQGLKWMGWINTYTGEPTYTDDFK GRFAFSLDTSVSTAYLQISSLKADDTAVYFCARGGFGSSYWYFDVWGQGSLVT VSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVE | Trop2 antibody scFab |
| 162 | QVQLQQSGSELKKPGASVKVSCKASGYTFTNYGMNWVKQAPGQGLKWMG WINTYTGEPTYTDDFKGRFAFSLDTSVSTAYLQISSLKADDTAVYFCARGGFGS SYWYFDVWGQGSLVTVSSASTKGPSVFPLAPSSKSTSGGTAALGCLVKDYFPE PVTVSWNSGALTSGVHTFPAVLQSSGLYSLSSVVTVPSSSLGTQTYICNVNHKP SNTKVDKKVE | Trop2 antibody Fab VH-CH1 |
| 163 | DIQLTQSPSFLSASVGDRVTITCKASQDVGSAVVWYQQKPGKAPKLLIYWASTR HTGVPSRFSGSGSGTEFTLTISSLQPEDFATYFCQQFTSYPTFGQGTKLEIKRTVA APSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGECGGG GSEGKSSGSGSESKSTEGKSSGSGSESKSTGGGGSQVQLVQSGAEVKKPGASV KVSCKASGYSFTGYYMHWVRQAPGQGLEWIGRINPYNGATTYSQNFKDRAT MTVDTSISTAYMELSRLRSDDTAVYYCVPTGMGYFLMDYWGQGTLVTVSSAS TKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKVE | PDL1 antibody scFab |
| 164 | QVQLVQSGAEVKKPGASVKVSCKASGYSFTGYYMHWVRQAPGQGLEWIGRI NPYNGATTYSQNFKDRATMTVDTSISTAYMELSRLRSDDTAVYYCVPTGMGYF LMDYWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNT KVDKKVE | PDL1 antibody Fab VH-CH1 |
| 165 | PKSCDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKC KVSNKALPAPIEKTISKAKGQPREPQVCTLPPSREEMTKNQVSLSCAVKGFYPS DIAVEWESNGQPENNYKTTPPVLDSDGSFFLVSKLTVDKSRWQQGNVFSCSVM HEALHNHYTQKSLSLSPGK | Second Fc peptide segment |
| 166 | PKSCDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKC KVSNKALPAPIEKTISKAKGQPREPQVYTLPPCREEMTKNQVSLWCLVKGFYPS DIAVEWESNGQPENNYKTTPPVLDSDGSFFLYSKLTVDKSRWQQGNVFSCSVM HEALHNHYTQKSLSLSPGK | Corresponding first Fc peptide segment when CD3 binding region is a Fab or scFab structure |

Hereinafter, the present disclosure will be described in detail by way of examples. In the examples or test examples, the experimental methods without specifying the specific conditions were carried out according to conventional conditions.

Hereinafter, the scheme of the present disclosure will be explained in conjunction with examples. Those skilled in the art will understand that the following examples are only used to illustrate the present disclosure and should not be considered as limiting the scope of the present disclosure. Where specific techniques or conditions are not specified in the examples, they are performed according to techniques or conditions described in the literature in the art or according to the product specification. The reagents or instruments used without specifying the manufacturer are all conventional products that can be obtained commercially.

### Example 1: Acquisition and production of an affinity-reduced antibody

During the affinity maturation process of natural antibodies, somatic hypermutations are mainly concentrated in the CDR region. Generally, single-point saturation mutagenesis is performed at each site in the CDR region through *in vitro* experiments to obtain sufficient mutation diversity without destroying the protein structure. This approach can achieve in vitro reproduction that is most similar to the somatic hypermutation of natural antibodies in vivo. The above method is generally used for affinity maturation (enhancement) of antibodies. With reference to this technical route, the present disclosure, conversely, reduces the affinity of CD3 antibodies.

The single-point saturation mutagenesis was performed on each amino acid site in the CDR region to construct an unbiased single-point saturation mutagenesis plasmid library of the parental antibody. Mutation hotspots with weakened specific binding to the antigen were screened out, and these hotspots were then combined for further screening to obtain candidate antibody mutation sequences.

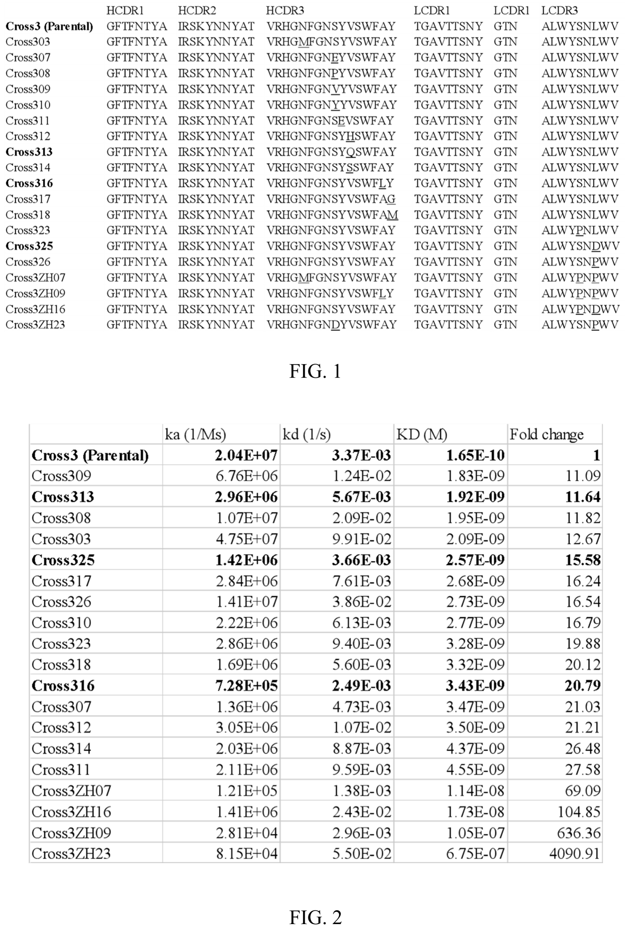

Through this technical route and after a large number of screening tests, the affinity of the parental antibody Cross3 (the amino acid sequence of the heavy chain variable region is as set forth in SEQ ID NO: 15, and the amino acid sequence of the light chain variable region is as set forth in SEQ ID NO: 16) was reduced, and the following low-affinity CD3 monoclonal antibodies were obtained: Cross303, Cross307, Cross308, Cross309, Cross310, Cross311, Cross312, Cross313, Cross314, Cross316, Cross317, Cross318, Cross323, Cross325, Cross326, Cross3ZH07, Cross3ZH09, Cross3ZH16, and Cross3ZH23. The amino acid sequences of the CDR region and the heavy and light chain amino acid sequences of the above-described low-affinity CD3 monoclonal antibody were shown in Table 1. The amino acid mutations in the CDR region compared with the parental antibody Cross3 were shown in FIG. 1.

The specific experimental operations for the production of the CD3 monoclonal antibodies were as follows. (1) ExpiCHO cells (purchased from Thermo Fisher) were cultured using ExpiCHO Expression Medium (purchased from Thermo Fisher), and the cell concentration was adjusted to 6×10⁶/mL to obtain ExpiCHO cell solution. (2) The pcDNA3.4 vectors containing the heavy chain and light chain of the CD3 antibody (for Cross3, heavy chain amino acid sequence is as set forth in SEQ ID NO: 35, and light chain amino acid sequence thereof is as set forth in SEQ ID NO: 33; for Cross303, heavy chain amino acid sequence is as set forth in SEQ ID NO: 37, and light chain amino acid sequence thereof is as set forth in SEQ ID NO: 33; for Cross307, heavy chain amino acid sequence is as set forth in SEQ ID NO: 38, and light chain amino acid sequence thereof is as set forth in SEQ ID NO: 33; for Cross308, heavy chain amino acid sequence is as set forth in SEQ ID NO: 39, and light chain amino acid sequence thereof is as set forth in SEQ ID NO: 33; for Cross309, heavy chain amino acid sequence is as set forth in SEQ ID NO: 40, and light chain amino acid sequence thereof is as set forth in SEQ ID NO: 33; for Cross310, heavy chain amino acid sequence is as set forth in SEQ ID NO: 41, and light chain amino acid sequence thereof is as set forth in SEQ ID NO: 33; for Cross311, heavy chain amino acid sequence is as set forth in SEQ ID NO: 42, and light chain amino acid sequence thereof is as set forth in SEQ ID NO: 33; for Cross312, heavy chain amino acid sequence is as set forth in SEQ ID NO: 43, and light chain amino acid sequence is as set forth in SEQ ID NO: 33; for Cross313, heavy chain amino acid sequence is as set forth in SEQ ID NO: 32, and light chain amino acid sequence is as set forth in SEQ ID NO: 33; for Cross314, heavy chain amino acid sequence is as set forth in SEQ ID NO: 44, and light chain amino acid sequence is as set forth in SEQ ID NO: 33; for Cross316, heavy chain amino acid sequence is as set forth in SEQ ID NO: 34, and light chain amino acid sequence is as set forth in SEQ ID NO: 33; for Cross317, heavy chain amino acid sequence is as set forth in SEQ ID NO: 45, and light chain amino acid sequence is as set forth in SEQ ID NO: 33; for Cross318, heavy chain amino acid sequence is as set forth in SEQ ID NO: 46, and light chain amino acid sequence is as set forth in SEQ ID NO: 33; for Cross323, heavy chain amino acid sequence is as set forth in SEQ ID NO: 45, and light chain amino acid sequence is as set forth in SEQ ID NO: 47; for Cross325, heavy chain amino acid sequence is as set forth in SEQ ID NO: 45, and light chain amino acid sequence thereof is as set forth in SEQ ID NO: 36; and for Cross326, heavy chain amino acid sequence is as set forth in SEQ ID NO: 46, and light chain amino acid sequence thereof is as set forth in SEQ ID NO: 48) (Synthesized by Nanjing GenScript) were added to 2 mL of OptiSFM medium (purchased from Thermo Fisher) at a ratio of 1: 1 to obtain solution a. (3) 160 µL of ExpiFectamine CHO transfection reagent (purchased from Thermo Fisher) was added to 2 mL of OptiSFM medium (purchased from Thermo Fisher) to obtain solution b. (4) The solution a and the solution b were then mixed to obtain a transfection mixture, and the entire transfection mixture was added to 50 mL of the ExpiCHO cell solution within 5 minutes. (5) After culturing at 37°C and 5% CO₂ for 1 day, 8 mL of Feed and 300 µL of Enhancer (purchased from Thermo Fisher) were added, and the cells were transferred to 32°C and 5% CO₂ and cultured for 9 days, with 8 mL of Feed added on day 5. The culture supernatant was harvested. (6) The target antibody was obtained by affinity purification from the culture supernatant using a Protein A purification column (purchased from NanoMicro).

The specific experimental operations for the production of bispecific antibodies were as follows. (1) ExpiCHO cells (purchased from Thermo Fisher) were cultured using ExpiCHO Expression Medium (purchased from Thermo Fisher), and the cell concentration was adjusted to 6×10⁶/mL to obtain ExpiCHO cell solution. (2) The pcDNA3.4 vectors containing CD3 antibody, Trop2 antibody heavy chain, and Trop2 antibody (Cross3×Trop2 contains CD3 antibody as set forth in SEQ ID NO: 52, Trop2 antibody heavy chain having an amino acid sequence as set forth in SEQ ID NO: 69, and Trop2 antibody light chain having an amino acid sequence as set forth in SEQ ID NO: 70; Cross303×Trop2 contains CD3 antibody as set forth in SEQ ID NO: 53, Trop2 antibody heavy chain having an amino acid sequence as set forth in SEQ ID NO: 69, and Trop2 antibody light chain having an amino acid sequence as set forth in SEQ ID NO: 70; Cross307×Trop2 contains CD3 antibody set forth in SEQ ID NO: 54, Trop2 antibody heavy chain having an amino acid sequence as set forth in SEQ ID NO: 69, and Trop2 antibody light chain having an amino acid sequence set forth in SEQ ID NO: 70; Cross308×Trop2 contains CD3 antibody as set forth in SEQ ID NO: 55, Trop2 antibody heavy chain having an amino acid sequence as set forth in SEQ ID NO: 69, and Trop2 antibody having an amino acid sequence as set forth in SEQ ID NO: 70; Cross309×Trop2 contains CD3 antibody as set forth in SEQ ID NO: 56, Trop2 antibody heavy chain having an amino acid sequence as set forth in SEQ ID NO: 69, and Trop2 antibody light chain having an amino acid sequence as set forth in SEQ ID NO: 70; Cross310×Trop2 contains CD3 antibody as set forth in SEQ ID NO: 57, Trop2 antibody heavy chain having an amino acid sequence as set forth in SEQ ID NO: 69, and Trop2 antibody light chain having an amino acid sequence as set forth in SEQ ID NO: 70; Cross311×Trop2 contains CD3 antibody as set forth in SEQ ID NO: 58, Trop2 antibody heavy chain having an amino acid sequence as set forth in SEQ ID NO: 69, and Trop2 antibody light chain having an amino acid sequence as set forth in SEQ ID NO: 70; Cross312×Trop2 contains CD3 antibody as set forth in SEQ ID NO: 59, Trop2 antibody heavy chain having an amino acid sequence as set forth in SEQ ID NO: 69, and Trop2 antibody light chain having an amino acid sequence as set forth in SEQ ID NO: 70; Cross313×Trop2 contains CD3 antibody as set forth in SEQ ID NO: 49, Trop2 antibody heavy chain having an amino acid sequence as set forth in SEQ ID NO: 69, and Trop2 antibody light chain having an amino acid sequence as set forth in SEQ ID NO: 70; Cross314×Trop2 contains CD3 antibody as set forth in SEQ ID NO: 60, Trop2 antibody heavy chain having an amino acid sequence as set forth in SEQ ID NO: 69, and Trop2 antibody light chain having an amino acid sequence as set forth in SEQ ID NO: 70; Cross316×Trop2 contains CD3 antibody as set forth in SEQ ID NO: 50, Trop2 antibody heavy chain having an amino acid sequence as set forth in SEQ ID NO: 69, and Trop2 antibody light chain having an amino acid sequence as set forth in SEQ ID NO: 70; Cross317×Trop2 contains CD3 antibody as set forth in SEQ ID NO: 61, Trop2 antibody heavy chain having an amino acid sequence as set forth in SEQ ID NO: 69, and Trop2 antibody light chain having an amino acid sequence as set forth in SEQ ID NO: 70; Cross318×Trop2 contains CD3 antibody as set forth in SEQ ID NO: 62, Trop2 antibody heavy chain having an amino acid sequence as set forth in SEQ ID NO: 69, and Trop2 antibody light chain having an amino acid sequence as set forth in SEQ ID NO: 70; Cross323×Trop2 contains CD3 antibody as set forth in SEQ ID NO: 63, Trop2 antibody heavy chain having an amino acid sequence as set forth in SEQ ID NO: 69, and Trop2 antibody light chain having an amino acid sequence as set forth in SEQ ID NO: 70; Cross325×Trop2 contains CD3 antibody as set forth in SEQ ID NO: 51, Trop2 antibody heavy chain having an amino acid sequence as set forth in SEQ ID NO: 69, and Trop2 antibody light chain having an amino acid sequence as set forth in SEQ ID NO: 70; Cross326×Trop2 contains CD3 antibody as set forth in SEQ ID NO: 64, Trop2 antibody heavy chain having an amino acid sequence as set forth in SEQ ID NO: 69, and Trop2 antibody light chain having an amino acid sequence as set forth in SEQ ID NO: 70) (synthesized by Nanjing GenScript), or the pcDNA3.4 vectors containing CD3 antibody, PDL1 antibody heavy chain, and PDL1 antibody light chain (Cross3×PDL1 contains CD3 antibody as set forth in SEQ ID NO: 52, PDL1 antibody heavy chain having an amino acid sequence as set forth in SEQ ID NO: 71, and PDL1 antibody light chain having an amino acid sequence as set forth in SEQ ID NO: 72; Cross303×PDL1 contains CD3 antibody as set forth in SEQ ID NO: 53, PDL1 antibody heavy chain having an amino acid sequence as set forth in SEQ ID NO: 71, and PDL1 antibody light chain having an amino acid sequence as set forth in SEQ ID NO: 72; Cross307×PDL1 contains CD3 antibody as set forth in SEQ ID NO: 54, PDL1 antibody heavy chain having an amino acid sequence as set forth in SEQ ID NO: 71, and PDL1 antibody light chain having an amino acid sequence as set forth in SEQ ID NO: 72; Cross308×PDL1 contains CD3 antibody as set forth in SEQ ID NO: 55, PDL1 antibody heavy chain having an amino acid sequence as set forth in SEQ ID NO: 71, and PDL1 antibody light chain having an amino acid sequence as set forth in SEQ ID NO: 72; Cross309×PDL1 contains CD3 antibody as set forth in SEQ ID NO: 56, PDL1 antibody heavy chain having an amino acid sequence as set forth in SEQ ID NO: 71, and PDL1 antibody light chain having an amino acid sequence as set forth in SEQ ID NO: 72; Cross310×PDL1 contains CD3 antibody as set forth in SEQ ID NO: 57, PDL1 antibody heavy chain having an amino acid sequence as set forth in SEQ ID NO: 71, and PDL1 antibody light chain having an amino acid sequence as set forth in SEQ ID NO: 72; Cross311×PDL1 contains CD3 antibody as set forth in SEQ ID NO: 58, PDL1 antibody heavy chain having an amino acid sequence as set forth in SEQ ID NO: 71, and PDL1 antibody light chain having an amino acid sequence as set forth in SEQ ID NO: 72; Cross312×PDL1 contains CD3 antibody as set forth in SEQ ID NO: 59, PDL1 antibody heavy chain having an amino acid sequence as set forth in SEQ ID NO: 71, and PDL1 antibody light chain having an amino acid sequence as set forth in SEQ ID NO: 72; Cross313×PDL1 contains CD3 antibody as set forth in SEQ ID NO: 49, PDL1 antibody heavy chain having an amino acid sequence as set forth in SEQ ID NO: 71, and PDL1 antibody light chain having an amino acid sequence as set forth in SEQ ID NO: 72; Cross314×PDL1 contains CD3 antibody as set forth in SEQ ID NO: 60, PDL1 antibody heavy chain having an amino acid sequence as set forth in SEQ ID NO: 71, and PDL1 antibody light chain having an amino acid sequence as set forth in SEQ ID NO: 72; Cross316×PDL1 contains CD3 antibody as set forth in SEQ ID NO: 50, PDL1 antibody heavy chain having an amino acid sequence as set forth in SEQ ID NO: 71, and PDL1 antibody light chain having an amino acid sequence as set forth in SEQ ID NO: 72; Cross317×PDL1 contains CD3 antibody as set forth in SEQ ID NO: 61, PDL1 antibody heavy chain having an amino acid sequence as set forth in SEQ ID NO: 71, and PDL1 antibody light chain having an amino acid sequence as set forth in SEQ ID NO: 72; Cross318×PDL1 contains CD3 antibody as set forth in SEQ ID NO: 62, PDL1 antibody heavy chain having an amino acid sequence as set forth in SEQ ID NO: 71, and PDL1 antibody light chain having an amino acid sequence as set forth in SEQ ID NO: 72; Cross323×PDL1 contains CD3 antibody as set forth in SEQ ID NO: 63, PDL1 antibody heavy chain having an amino acid sequence as set forth in SEQ ID NO: 71, and PDL1 antibody light chain having an amino acid sequence as set forth in SEQ ID NO: 72; Cross325×PDL1 contains CD3 antibody as set forth in SEQ ID NO: 51, PDL1 antibody heavy chain having an amino acid sequence as set forth in SEQ ID NO: 71, and PDL1 antibody light chain having an amino acid sequence as set forth in SEQ ID NO: 72; Cross326×PDL1 contains CD3 antibody as set forth in SEQ ID NO: 64, PDL1 antibody heavy chain having an amino acid sequence as set forth in SEQ ID NO: 71, and PDL1 antibody light chain having an amino acid sequence as set forth in SEQ ID NO: 72; Cross3ZH07×PDL1 contains CD3 antibody as set forth in SEQ ID NO: 65, PDL1 antibody heavy chain having an amino acid sequence set forth in SEQ ID NO: 71, and PDL1 antibody light chain having an amino acid sequence as set forth in SEQ ID NO: 72; Cross3ZH09×PDL1 contains CD3 antibody as set forth in SEQ ID NO: 66, PDL1 antibody heavy chain having an amino acid sequence as set forth in SEQ ID NO: 71, and PDL1 antibody light chain having an amino acid sequence as set forth in SEQ ID NO: 72; Cross3ZH16×PDL1 contains CD3 antibody as set forth in SEQ ID NO: 67, PDL1 antibody heavy chain having an amino acid sequence as set forth in SEQ ID NO: 71, and PDL1 antibody light chain having an amino acid sequence as set forth in SEQ ID NO: 72; Cross3ZH23×PDL1 contains CD3 antibody as set forth in SEQ ID NO: 68, PDL1 antibody heavy chain having an amino acid sequence as set forth in SEQ ID NO: 71, and PDL1 antibody light chain having an amino acid sequence as set forth in SEQ ID NO: 72) (synthesized by Nanjing GenScript) were added to 2 mL of OptiSFM medium (purchased from Thermo Fisher) at a ratio of 1: 1: 1 to obtain solution a. (3) 160 µL of ExpiFectamine CHO transfection reagent (purchased from Thermo Fisher) was added to 2 mL of OptiSFM medium (purchased from Thermo Fisher) to obtain solution b. (4) The solution a and the solution b were then mixed to obtain a transfection mixture, and the entire transfection mixture was added to 50 mL of the ExpiCHO cell solution within 5 minutes. (5) After culturing at 37°C and 5% CO₂ for 1 day, 8 mL of Feed and 300 µL of Enhancer (purchased from Thermo Fisher) were added, and the cells were transferred to 32°C and 5% CO₂ and cultured for 9 days, with 8 mL of Feed added on day 5. The culture supernatant was harvested. (6) The target CD3×Trop2 or CD3×PDL1 antibodies were obtained by affinity purification from the culture supernatant using a Protein A purification column (purchased from NanoMicro). The antibody configuration was shown in FIG. 5.

### Example 2: Antibody affinity assay

Biacore is a method for analyzing biomolecular interactions based on the principle of optical surface plasmon resonance (SPR). It can not only detect the specific binding between antigens and antibodies, but also obtain intermolecular binding rate constant (ka), dissociation rate constant (kd), equilibrium dissociation constant (KD), etc., which are very important data in drug development, thereby enabling calculation of antibody affinity.

In a Biacore 8K (Cytiva) system, the antibody was diluted to 10 µg/mL in HBS-EP running buffer and coupled to a CM5 chip (Cytiva) at a flow rate of 10 µL/min. At a flow rate of 30 µL/min, the kinetics and affinity data of the binding of CD3 E&D antigens to the low-affinity CD3 monoclonal antibody of Example 1 of the present disclosure were detected, with the association time set to 120 s and the dissociation time set to 800 s. The results of the antibody affinity assay were shown in FIG. 2.

### Example 3: CD3 monoclonal antibody ELISA binding assay

In this example, the binding properties of the parental antibody Cross3 and the CD3 affinity-reduced antibody (the production method and the amino acid sequences of the heavy and light chains were shown in Example 1) were detected by ELISA. The CD3E&D protein was coated onto a 96-well plate by the inventors, and the strength of the signal after the addition of the antibody was used to determine the binding properties of the antibody and CD3E&D.

CD3E&D protein (purchased from Acro) was diluted to 2 µg/ml with PBS buffer, added to a 96-well plate at a volume of 100 µL/well, and placed at 4°C overnight. The PBS buffer in the 96-well plate was aspirated, and the plate was washed 6 times with PBST (pH 7.2 PBS containing 0.1% Tween 20) buffer. Then, 200 µL/well PBS/10% BSA was added and incubated at 37°C for 2 h for blocking. The blocking solution was removed, and the plate was washed six times with PBST. Then, the to-be-tested antibodies serially diluted with PBST/0.05% BSA, Cross3, Cross303, Cross307, Cross308, Cross309, Cross310, Cross311, Cross312, Cross313, Cross314, Cross316, Cross317, Cross318, Cross323, Cross325, Cross326, and control IgG1 (purchased from Bio-Tech) were added at 100 µL/well, and incubated at 37°C for 1 h. The reaction system was removed, and the plate was washed 6 times with PBST. Then, the cells were incubated at 37°C for 1 h with HRP (horseradish peroxidase)-labeled anti-human secondary antibody (Fab specific) (purchased from Sigma) serially diluted with PBST/0.05% BSA at 100 µL/well. The plate was washed six times with PBST, TMB (tetramethylbenzidine) was added at 80 µL/well and incubated at room temperature for 3 min. 4 M sulfuric acid was added at 80 µL/well to terminate the reaction. The absorbance was read at 450 nm using a microplate reader.

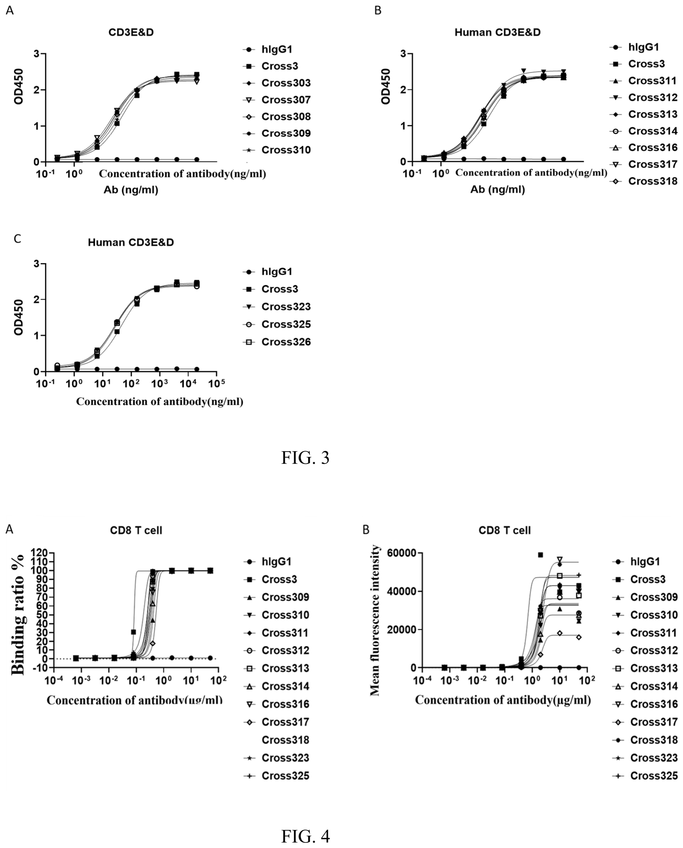

The results were shown in FIG. 3, which indicate that all CD3 antibodies of the present disclosure can bind to CD3 E&D. However, due to the limited resolution of the ELISA assay, the ELISA results cannot reflect the differences in affinity among various CD3 monoclonal antibodies.

### Example 4: CD3 monoclonal antibody flow cytometry binding assay

PBMCs were diluted with PBS to 2×10⁶/mL and added to a 1.5 ml EP tube at a volume of 100 µL/tube, 10 µL/tube of goat serum was added thereto and incubated at 4°C for 30 min for blocking. The to-be-tested antibodies serially diluted, Cross3, Cross303, Cross307, Cross308, Cross309, Cross310, Cross311, Cross312, Cross313, Cross314, Cross316, Cross317, Cross318, Cross323, Cross325, Cross326 (the production method and the amino acid sequences of the heavy and light chains of the antibody Cross3 and the CD3 affinity-reduced antibody were shown in Example 1), and control IgG1 (purchased from Bio-Tech) were added and incubate at 4°C for 30 min. 1 mL of PBS was added to the EP tube, followed by centrifugation at 3500 rpm for 5 min at 4°C. The supernatant was discarded, and the cells were washed again with PBS. After centrifugation, the supernatant was discarded, and the cells were resuspended with 100 µl/tube PBS. 1 µl/tube Alexa-647-labeled goat anti-human secondary antibody (purchased from Jackson lab) and 0.5 µl/tube PerCP-Cy5.5-labeled anti-human CD8 antibody were added and incubated at 4°C in the dark for 30 min. The cells were washed twice with PBS, and the supernatant was discarded after centrifugation. The cells were resuspended with 200 µL/tube of PBS and assayed using the flow cytometry.

The results were shown in FIG. 4, which show that the CD3 antibodies, Cross309, Cross310, Cross311, Cross312, Cross313, Cross314, Cross316, Cross317, Cross318, Cross323, and Cross325 of the present disclosure can bind to CD8 T cells with reduced CD3 affinity, and the CD3 antibody binding ability is weaker than that of the parental antibody Cross3.

### Example 5: CD3×Trop2 and CD3×PDL1 bispecific antibody ELISA binding assay

In this example, the binding properties of the CD3×Trop2 and CD3×PDL1 bispecific antibody (the production method and the amino acid sequences of the heavy and light chains were shown in Example 1) were detected by using ELISA. The CD3E&D, Trop2, or PDL1 protein was coated onto 96-well plates by inventors, and the strength of the signal after the addition of the antibody was used to determine the binding properties of the antibody and the corresponding protein.
(1) CD3E&D protein (purchased from Acro) was diluted to 2 µg/ml with PBS buffer, added to a 96-well plate at a volume of 100 µL/well, and placed at 4°C overnight. The PBS buffer in the 96-well plate was aspirated, and the plate was washed 6 times with PBST (pH 7.2 PBS containing 0.1% Tween 20) buffer. Then, 200 µL/well PBS/10% BSA was added and incubated at 37°C for 2 h for blocking. The blocking solution was removed, and the plate was washed 6 times with PBST. Then, the to-be-tested bispecific antibodies serially diluted with PBST/0.05% BSA, Cross3×Trop2, Cross303×Trop2, Cross307×Trop2, Cross308×Trop2, Cross309×Trop2, Cross310×Trop2, Cross311×Trop2, Cross312×Trop2, Cross313×Trop2, Cross314×Trop2, Cross316×Trop2, Cross317×Trop2, Cross318×Trop2, Cross323×Trop2, Cross325×Trop2, Cross326×Trop2, Cross3×PDL1, Cross303×PDL1, Cross307×PDL1, Cross308×PDL1, Cross309×PDL1, Cross310×PDL1, Cross311×PDL1, Cross312×PDL1, Cross313×PDL1, Cross314×PDL1, Cross316×PDL1, Cross317xPDL1, Cross318×PDL1, Cross323×PDL1, Cross325×PDL1, Cross326×PDL1, and control IgG1LALA (purchased from Bio-Tech) were added at 100 µL/well, and incubated at 37°C for 1 h. The reaction system was removed, and the plate was washed 6 times with PBST. Then, the cells were incubated at 37°C for 1 h with HRP (horseradish peroxidase)-labeled anti-human secondary antibody (Fab specific) (purchased from Sigma) diluted with PBST/0.05% BSA at 100 µL/well. After the plate was washed six times with PBST, 80 µL/well TMB (tetramethylbenzidine) was added and incubated at room temperature for 3 min. 4 M sulfuric acid was added at 80 µL/well to terminate the reaction. The absorbance was read at 450 nm using a microplate reader.

As shown in FIG. 6, the CD3×Trop2 antibody of the present disclosure can bind to CD3E&D. Compared with the parental Cross3×Trop2 bispecific antibody, the binding of the affinity-reduced CD3 bispecific antibody to CD3E&D protein is weakened, which is consistent with expectations.

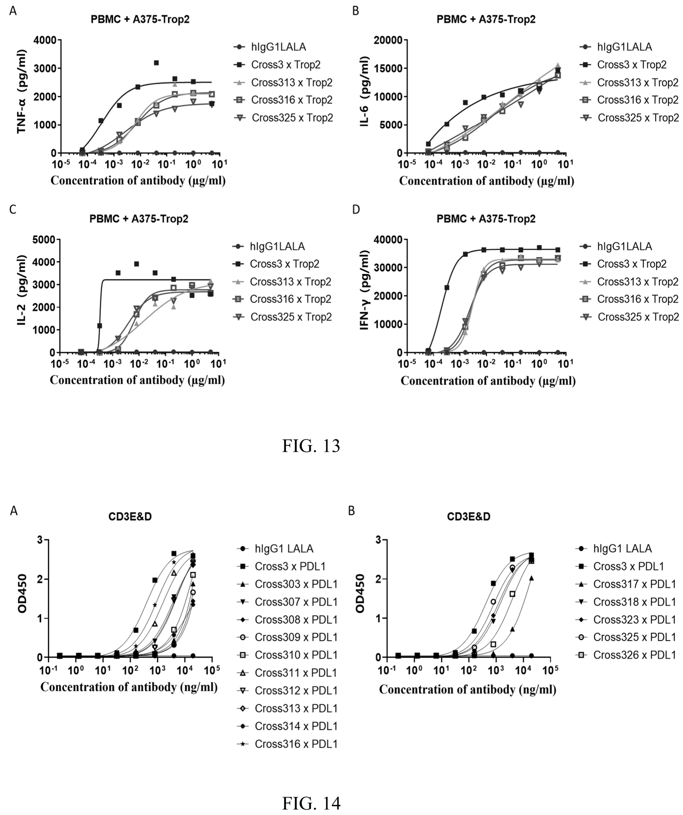

As shown in FIG. 14, the CD3×PDL1 antibody of the present disclosure can bind to CD3E&D. Compared with the parent Cross3×PDL1 bispecific antibody, the binding of the affinity-reduced CD3 bispecific antibody to CD3E&D protein is weakened, which is consistent with expectations.

(2) Trop2 protein (purchased from Acro) was diluted to 2 µg/ml with PBS buffer, added to a 96-well plate at a volume of 100 µL/well, and placed at 4°C overnight. The PBS buffer in the 96-well plate was aspirated, and the plate was washed 6 times with PBST (pH 7.2 PBS containing 0.1% Tween 20) buffer. Then, 200 µL/well PBS/10% BSA was added and incubated at 37°C for 2 h for blocking. The blocking solution was removed, and the plate was washed six times with PBST. Then, the to-be-tested bispecific antibodies, Cross3×Trop2, Cross303×Trop2, Cross307×Trop2, Cross308×Trop2, Cross309×Trop2, Cross310×Trop2, Cross311×Trop2, Cross312×Trop2, Cross313×Trop2, Cross314×Trop2, Cross316×Trop2, Cross317×Trop2, Cross318×Trop2, Cross323×Trop2, Cross325×Trop2, Cross326×Trop2, and control IgG1LALA (purchased from Bio-Tech), serially diluted with PBST/0.05% BSA, were added at 100 µL/well and incubated at 37°C for 1 h. The reaction system was removed, and the plate was washed 6 times with PBST. Then, HRP (horseradish peroxidase)-labeled anti-human secondary antibody (Fab specific) (purchased from Sigma) diluted with PBST/0.05% BSA at 100 µL/well. After the plate was washed six times with PBST, TMB (tetramethylbenzidine) was added at 80 µL/well, and incubated at room temperature for 3 min. 4 M sulfuric acid was added at 80 µL/well to terminate the reaction. The absorbance was read at 450 nm using a microplate reader.

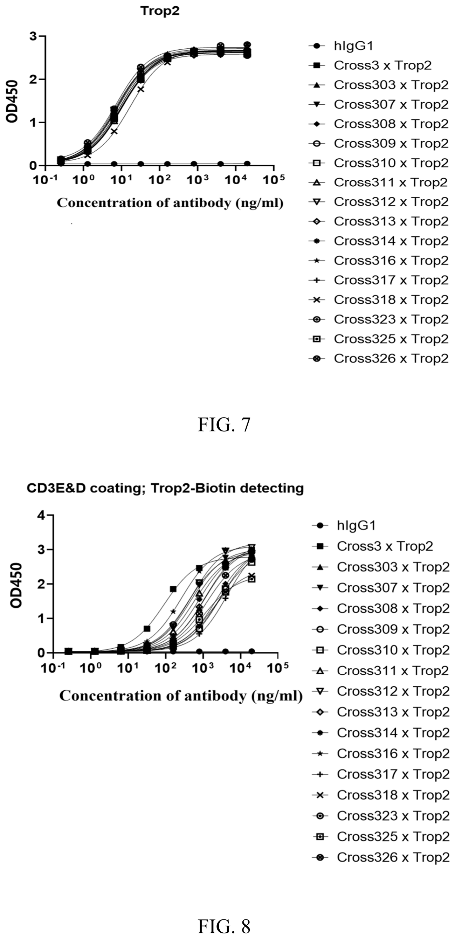

As shown in FIG. 7, it indicates that the CD3×Trop2 antibody of the present disclosure can bind to the Trop2 protein, and all bispecific antibodies exhibit similar binding to the Trop2 protein.

(3) PDL1 protein (purchased from Acro) was diluted to 2 µg/ml with PBS buffer, added to a 96-well plate at a volume of 100 µL/well, and placed at 4°C overnight. The PBS buffer in the 96-well plate was aspirated, and the plate was washed 6 times with PBST (pH 7.2 PBS containing 0.1% Tween 20) buffer. Then, PBS/10% BSA was added at 200 µL/well and incubated at 37°C for 2 h for blocking. The blocking solution was removed, and the plate was washed six times with PBST. Then, the to-be-tested bispecific antibodies serially diluted with PBST/0.05% BSA, Cross3×PDL1, Cross303×PDL1, Cross307×PDL1, Cross308×PDL1, Cross309×PDL1, Cross310×PDL1, Cross311×PDL1, Cross312×PDL1, Cross313×PDL1, Cross314×PDL1, Cross316×PDL1, Cross317×PDL1, Cross318×PDL1, Cross323×PDL1, Cross325×PDL1, Cross326×PDL1, and control IgG1LALA (purchased from Bio-Tech) were added at 100 µL/well, and incubated at 37°C for 1 h. The reaction system was removed, and the plate was washed 6 times with PBST. Then, the cells were incubated at 37°C for 1 h with HRP (horseradish peroxidase)-labeled anti-human secondary antibody (Fab specific) (purchased from Sigma) serially diluted with PBST/0.05% BSA at 100 µL/well. After the plate was washed six times with PBST, TMB (tetramethylbenzidine) was added at 80 µL/well and incubated at room temperature for 3 min. 4 M sulfuric acid was added at 80 µL/well to terminate the reaction. The absorbance was read at 450 nm using a microplate reader.

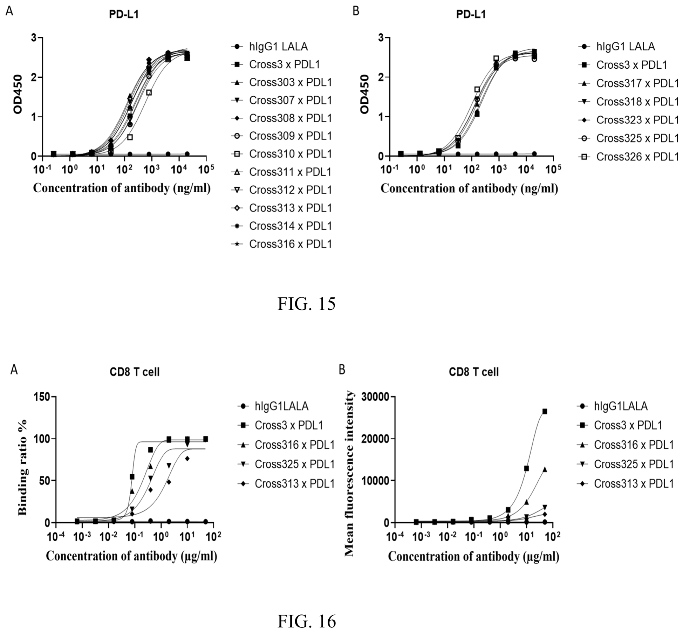

As shown in FIG. 15, it indicates that the CD3×PDL1 antibody of the present disclosure can bind to the PDL1 protein.

### Example 6: CD3×Trop2 antibody ELISA bridging assay

ELISA assay was used to detect the bridging binding properties of the CD3×Trop2 bispecific antibody (the production method and amino acid sequences of the heavy and light chains were shown in Example 1). CD3E&D antigen protein was coated onto a 96-well plate. After the antibody was added, detection was performed by using biotin-labeled Trop2 protein. The strength of the signal was used to determine the binding properties of the bispecific antibody bridging CD3E&D and Trop2 proteins.

CD3E&D protein (purchased from Acro) was diluted to 2 µg/ml with PBS buffer, added to a 96-well plate at a volume of 100 µl/well, and placed at 4°C overnight. The PBS buffer in the 96-well plate was aspirated, and the plate was washed 6 times with PBST (pH 7.2 PBS containing 0.1% Tween 20) buffer. Then, PBS/10% BSA was added at 200 µl/well, and incubated at 37°C for 2 h for blocking. The blocking solution was removed, and the plate was washed six times with PBST. Then, the to-be-tested bispecific antibodies serially diluted with PBST/0.05% BSA, Cross3×Trop2, Cross303×Trop2, Cross307×Trop2, Cross308×Trop2, Cross309×Trop2, Cross310×Trop2, Cross311×Trop2, Cross312×Trop2, Cross313×Trop2, Cross314×Trop2, Cross316×Trop2, Cross317×Trop2, Cross318×Trop2, Cross323×Trop2, Cross325×Trop2, Cross326×Trop2, and the control hIgG1LALA (purchased from Bio-Tech), were added at 100 µl/well, and incubated at 37°C for 1 h. The reaction system was removed, and the plate was washed 6 times with PBST. Trop2-Biotin protein diluted to an appropriate concentration was added and incubated at 37°C for 1 h. The reaction system was removed, and the plate was washed 6 times with PBST. The cells were incubated at 37°C for 1 h with HRP (horseradish peroxidase)-labeled Streptavidin secondary antibody (purchased from Southern Biotech) serially diluted with PBST/0.05% BSA at 100 µl/well. After the plate was washed six times with PBST, TMB (tetramethylbenzidine) was added at 80 µl/well, and incubated at room temperature for 3 min. 4 M sulfuric acid was added at 80 µl/well to terminate the reaction. The absorbance was read at 450 nm using a microplate reader.

The results were shown in FIG. 8, indicating that the bispecific antibodies of the present disclosure can bridge CD3E&D and Trop2 proteins, and the bridging ability of the parental Cross3×Trop2 antibody is stronger than that of the affinity-reduced CD3 bispecific antibodies, which is consistent with expectations.

### Example 7: Bispecific antibody flow cytometry binding assay

The flow cytometry experiment was used to detect the binding properties of the bispecific antibodies. The bispecific antibodies (the production method and amino acid sequences of heavy and light chains were shown in Example 1) were added to cells, and the strength of the signal after the addition of the antibody was measured.
(1) PBMCs were diluted to 2×10⁶/ml with PBS and added to a 1.5 ml EP tube at 100 µl/tube, goat serum was added thereto at 10 µl/tube. The cells were blocked at 4°C for 30 min. The serially diluted bispecific antibodies, Cross3×Trop2, Cross303×Trop2, Cross307×Trop2, Cross308×Trop2, Cross309×Trop2, Cross310×Trop2, Cross311×Trop2, Cross312×Trop2, Cross313×Trop2, Cross314×Trop2, Cross316×Trop2, Cross317×Trop2, Cross318×Trop2, Cross323×Trop2, Cross325×Trop2, Cross326×Trop2, Cross3×PDL1, Cross313×PDL1, Cross316×PDL1, Cross325×PDL1, and control hIgG1LALA (purchased from Bio-Tech) were added and incubated at 4°C for 30 min. 1 ml of PBS was added to the EP tube, followed by centrifugation at 3500 rpm for 5 min at 4°C. The supernatant was discarded, and the system was washed again with PBS. After centrifugation, the supernatant was discarded, and the cells were resuspended with PBS at100 µl/tube. 1 µl/tube Alexa-647-labeled goat anti-human IgG secondary antibody (purchased from Jackson lab) and 0.5 µl/tube PerCP-Cy5.5-labeled anti-human CD8 antibody were added and incubated at 4°C in the dark for 30 min. The cells were washed twice with PBS. After centrifugation, the supernatant was discarded. The cells were resuspended with 200 µl/tube of PBS and assay is performed by flow cytometry.

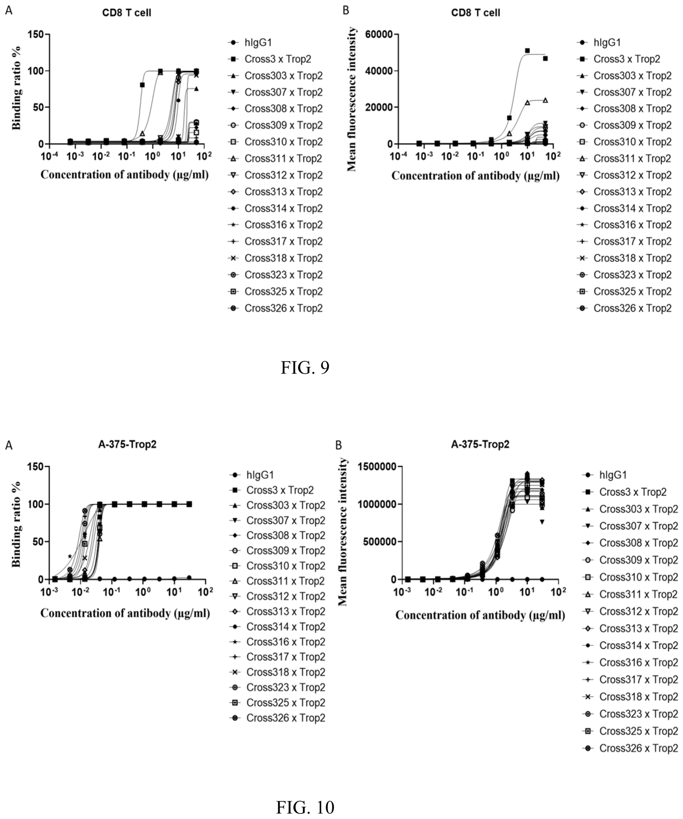

The results, as shown in FIG. 9, indicate that the CD3×Trop2 antibody of the present disclosure can bind to CD8 T cells, and compared with the parental CD3 antibody Cross3×Trop2, the ability of the CD3 affinity-reduced bispecific antibody to bind to T cells is weakened, which is consistent with expectations.

The results, as shown in FIG. 16, indicate that the Cross3×PDL1, Cross313×PDL1, Cross316×PDL1, and Cross325×PDL1 antibodies of the present disclosure can bind to CD8 T cells, and the binding ability of Cross313×PDL1, Cross316×PDL1, and Cross325xPDL1 to T cells is weaker than that of Cross3×PDL1, which is consistent with expectations.

(2) The tumor cells were diluted to 2×10⁶/ml with PBS and added to a 1.5ml EP tube at a volume of 100µl/tube, and goat serum was added thereto at 10µl/tube. The cells were blocked at 4°C for 30 min. The serially diluted bispecific antibodies, Cross3×Trop2, Cross303×Trop2, Cross307×Trop2, Cross308×Trop2, Cross309×Trop2, Cross310×Trop2, Cross311×Trop2, Cross312×Trop2, Cross313×Trop2, Cross314×Trop2, Cross316×Trop2, Cross317×Trop2, Cross318×Trop2, Cross323×Trop2, Cross325×Trop2, Cross326×Trop2, and Cross3×PDL1, Cross303×PDL1, Cross307×PDL1, Cross308×PDL1, Cross309×PDL1, Cross310×PDL1, Cross311×PDL1, Cross312×PDL1, Cross313×PDL1, Cross314×PDL1, Cross316×PDL1, Cross317×PDL1, Cross318×PDL1, Cross323×PDL1, Cross325×PDL1, Cross326×PDL1, and control hIgG1LALA (purchased from Bio-Tech) were added and incubated at 4°C for 30 min. 1 ml of PBS was added to the EP tube, followed by centrifugation at 3500 rpm for 5 min at 4°C. The supernatant was discarded, and the cells were washed again with PBS. After centrifugation, the supernatant was completely discarded, and the cells were resuspended with PBS at 100 µl/tube. Alexa-647-labeled goat anti-human IgG secondary antibody (purchased from Jackson lab) was added at 1 µl/tube and incubated at 4°C in the dark for 30 min. The cells were washed twice with PBS, and the supernatant was completely discarded after centrifugation. The cells were resuspended with PBS at 200 µl/tube and assay wasperformed using the flow cytometry.

The results, as shown in FIG. 10, indicate that the CD3×Trop2 antibodies of the present disclosure can bind to A-375-Trop2 melanoma cells. Based on the mean fluorescence intensity, all bispecific antibodies have similar binding abilities to A-375-Trop2 cells.

The results, as shown in FIG.S 17, 18, and 19, indicate that the CD3×PDL1 antibody of the present disclosure can bind to A-375 melanoma cells, HCT-15 colorectal cancer cells, and A549 lung cancer cells.

### Example 8: Experiment on bispecific antibody promoting the killing of tumor cells by PBMCs

The ability of bispecific antibodies to promote the killing of A-375 melanoma cells and HCT-15 colorectal cancer cells by PBMCs was detected.
(a) Complete RPMI-1450 medium was added to a 16-well RTCA plate at a volume of 50 µL/well, and the instrument was calibrated after loading.
(b) The tumor cells were diluted to 2×10⁵/mL with the complete RPMI-1450 medium and added separately to the RTCA plates obtained in step (1) at a volume of 50 µL/well, respectively. The cell index was then detected for 24 h using the xCELLigence RTCA MP instrument under the conditions of 37°C and 5% CO₂.
(c)The bispecific antibodies serially diluted with the complete RPMI-1450 medium, Cross3×Trop2, Cross303×Trop2, Cross307×Trop2, Cross308×Trop2, Cross309×Trop2, Cross310×Trop2, Cross311×Trop2, Cross312×Trop2, Cross313×Trop2, Cross314×Trop2, Cross316×Trop2, Cross317×Trop2, Cross318×Trop2, Cross323×Trop2, Cross325×Trop2, Cross326×Trop2, Cross3×PDL1, and Cross303×PD L1, Cross307×PDL1, Cross308×PDL1, Cross309×PDL1, Cross310×PDL1, Cross311×PDL1, Cross312×PDL1, Cross313×PDL1, Cross314×PDL1, Cross316×PDL1, Cross317×PDL1, Cross318×PDL1, Cross323×PDL1, Cross325×PDL1, Cross326×PDL1, Cross3ZH07×PDL1, Cross3ZH09×PDL1, Cross3ZH16×PDL1, Cross3ZH23×PDL1, and control hIgG1LALA (purchased from Bio-Tech) were added to the RTCA plate obtained in step (2) at a volume of 20 µl/well.

(4) PBMCs (purchased from Miaoshun Biotechnology) were diluted to 1.25×10⁶ cells/ml with the complete RPMI-1450 medium and added to the RTCA plate obtained in step (3) at a volume of 80 µl/well.
(5) The reaction system obtained in step (4) was incubated at 37°C and 5% CO₂, and the cell index was detected for 24 h by using an xCELLigence RTCA MP instrument.

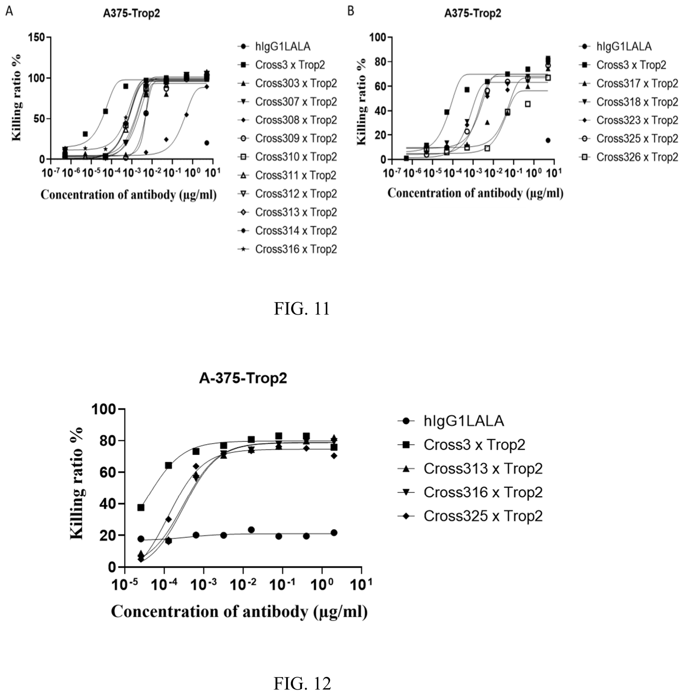

As shown in FIG. 11, the CD3×Trop2 antibody of the present disclosure promotes the killing of A-375-Trop2 melanoma cells by PBMCs. The effective dose of the Cross3×Trop2 bispecific antibody is lower than that of the affinity-reduced bispecific antibody. Although the effective doses of Cross313×Trop2, Cross316×Trop2, and Cross325×Trop2 are high, the maximum killing ratio is consistent with that of Cross3×Trop2, and all can achieve the highest killing.

As shown in FIG. 12, Cross3×Trop2, Cross313×Trop2, Cross316×Trop2, and Cross325×Trop2 promote the killing of A-375-Trop2 melanoma cells by PBMCs. Although the effective doses of Cross313×Trop2 and Cross316×Trop2 are high, the highest killing ratio is consistent with that of Cross3×Trop2, and both can achieve the highest killing.

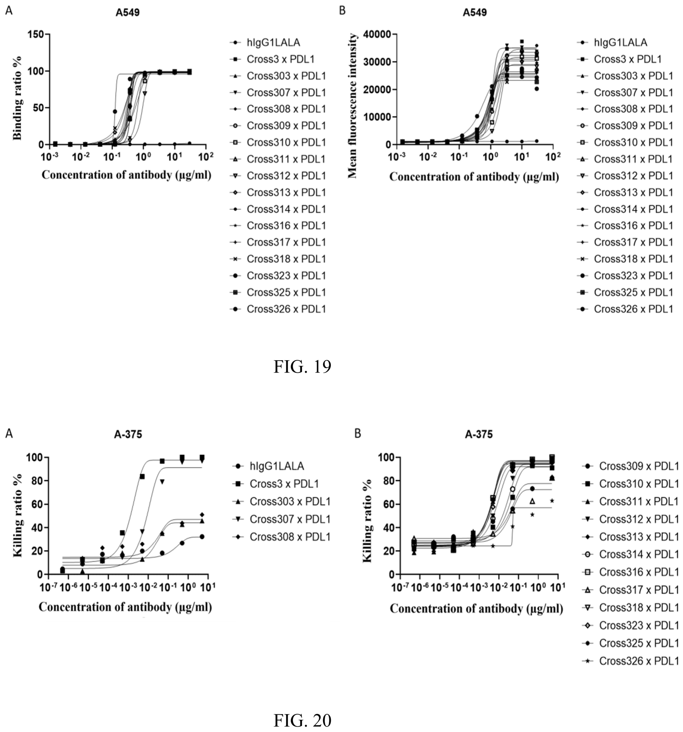

As shown in FIG. 20, CD3×PDL1 of the present disclosure promotes the killing of kill A-375 melanoma cells by PBMCs, and Cross313×PDL1, Cross316×PDL1, and the maximum killing of Cross325×PDL1 is superior to that of other bispecific antibodies.

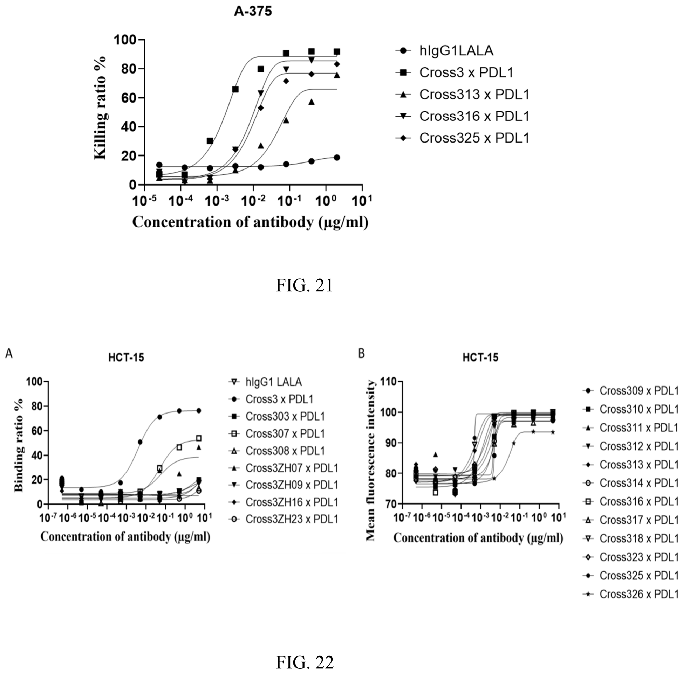

As shown in FIG. 21, Cross3×PDL1, Cross313×PDL1, Cross316×PDL1, and Cross325×PDL1 of the present disclosure promote PBMC to kill A-375 melanoma cells, and the maximum killing of Cross316×PDL1 is superior to that of Cross313×PDL1 and Cross325×PDL1.

As shown in FIG. 22, CD3×PDL1 of the present disclosure promotes the killing of HCT-15 colorectal cancer cells by PBMCs, and Cross313×PDL1, Cross316×PDL1, and Cross325×PDL1 can promote 100% killing of HCT-15 colorectal cancer cells by PBMCs.

### Example 9: Experiment on bispecific antibodies promoting cytokine secretion by PBMCs

The bispecific antibodies (the production method and amino acid sequences of heavy and light chains were shown in Example 1) were added to the co-incubation system of PBMCs and tumor cells. After 48 h of incubation, the culture supernatant was collected, and the cytokine content in the supernatant was detected to determine the characteristics of the bispecific antibody in inducing cytokine release.
(1) The tumor cells were diluted to 1×10⁵/ml with complete RPMI 1450 medium, added to a 96-well plate, and cultured in an incubator at 37°C and 5% CO₂ for 24 h.
(2) The bispecific antibodies Cross3×Trop2, Cross313×Trop2, Cross316×Trop2, Cross325×Trop2, Cross3×PDL1, Cross313×PDL1, Cross316×PDL1, Cross325×PDL1, and control hIgG1LALA (purchased from Bio-Bio) were serially diluted with complete RPMI 1450 medium and added to a 96-well plate at 20 µl/well.
(3) PBMCs (purchased from Saili Biotechnology) were diluted to 1.25×10⁶/ml with complete RPMI 1450 medium and added to a 96-well plate at 80 µl/well.
(4) The 96-well plate was incubated in an incubator at 37°C and 5% CO₂ for 48 h.
(5) The cells were centrifuged at 300g for 10 min at room temperature, and the cell culture supernatant was collected.
(6) The cytokine content in the supernatant was detected using CBA kit (purchased from BD).

As shown in FIG. 13, Cross3×Trop2, Cross313×Trop2, Cross316×Trop2, and Cross325×Trop2 of the present disclosure promote the secretion of pro-inflammatory cytokines TNF-α, IL-6, IFN-y, and IL-2 by PBMCs, and the ability of the Cross313×Trop2, Cross316×Trop2, and Cross325×Trop2 antibodies in promoting the secretion of pro-inflammatory cytokines is weaker than that of Cross3×Trop2.

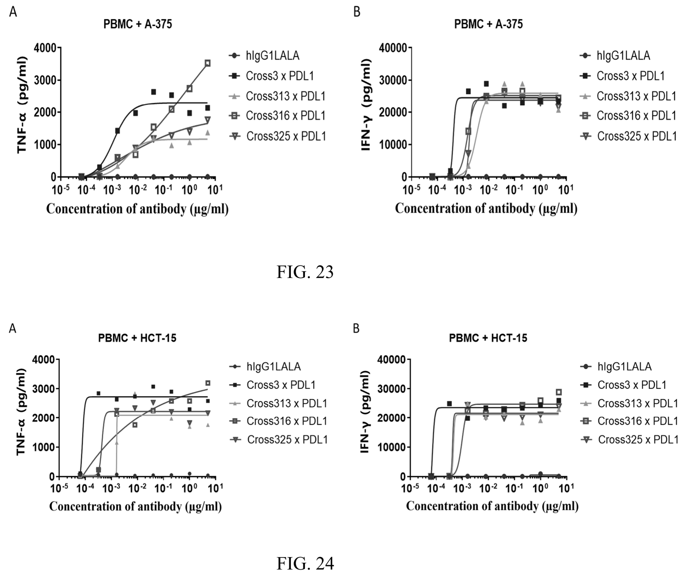

As shown in FIG. 23 and FIG. 24, Cross3×PDL1, Cross313×PDL1, Cross316×PDL1, and Cross325×PDL1 of the present disclosure promote the secretion of pro-inflammatory cytokines TNF-α and IFN-γ by PBMCs, and the ability of the Cross313×PDL1, Cross316×PDL1, and Cross325×PDL1 antibodies in promoting the secretion of pro-inflammatory cytokines is weaker than that of Cross3×PDL1.

In the description of the present specification, reference to the terms such as "one embodiment", "some embodiments", "an example", "a specific example", or "some examples" mean that the specific features, structures, materials, or characteristics described in connection with the embodiment or example are included in at least an embodiment or example of the present disclosure. In the present specification, the illustrative descriptions of the above terms do not necessarily refer to the same embodiment or example. Moreover, the specific features, structures, materials, or characteristics described may be combined in any one or more embodiments or examples in a suitable manner. Furthermore, where there is no mutual contradiction, those skilled in the art may combine and integrate different embodiments or examples and features of different embodiments or examples described in this specification.

Although embodiments of the present disclosure are illustrated and described above, it can be understood that the above embodiments are illustrative and should not be construed as limiting the present disclosure. Those skilled in the art can make changes, modifications, substitutions, and variations to the above embodiments within the scope of the present disclosure.

## Claims

1. An anti-CD3 antibody or antigen binding fragment thereof, comprising:
heavy chain variable region CDRs; and
light chain variable region CDRs, wherein:
the heavy chain variable region CDRs have each an amino acid sequence as set forth in one of SEQ ID NOs: 1 to 3, 7 to 8, and 74 to 86, or a conservative modification form thereof; and/or
the light chain variable region CDRs have each an amino acid sequence as set forth in one of SEQ ID NOs: 4 to 6, 9, and 87 to 92, or a conservative modification form thereof.

2. The antibody or antigen binding fragment thereof according to claim 1, wherein:
the heavy chain variable region CDRs have amino acid sequences as set forth in SEQ ID NOs: 1 to 2 or a conservative modification form thereof, and an amino acid sequence as set forth in one of SEQ ID NOs: 3, 7 to 8, and 74 to 86 or a conservative modification form thereof; and
the light chain variable region CDRs have amino acid sequences as set forth in SEQ ID NOs: 4 to 5 or a conservative modification form thereof, and an amino acid sequence as set forth in one of SEQ ID NOs: 6, 9, and 87 to 92 or a conservative modification form thereof.

3. The antibody or antigen binding fragment thereof according to claim 2, comprising:
a heavy chain variable region CDR1 having an amino acid sequence as set forth in SEQ ID NO: 1,
a heavy chain variable region CDR2 having an amino acid sequence as set forth in SEQ ID NO: 2,
a heavy chain variable region CDR3 having an amino acid sequence as set forth in one of SEQ ID NO: 3, SEQ ID NOs: 7 to 8, and SEQ ID NOs: 74 to 86;
a light chain variable region CDR1 having an amino acid sequence as set forth in SEQ ID NO: 4,
a light chain variable region CDR2 having an amino acid sequence as set forth in SEQ ID NO: 5, and
a light chain variable region CDR3 having an amino acid sequence as set forth in one of SEQ ID NOs: 6, 9, and 87 to 92.

4. The antibody or antigen binding fragment thereof according to claim 3, comprising any one of the following groups:
1) the heavy chain variable region CDR1, the heavy chain variable region CDR2, and the heavy chain variable region CDR3 as set forth in SEQ ID NOs: 1, 2, and 74, respectively, and the light chain variable region CDR1, the light chain variable region CDR2, and the light chain variable region CDR3 as set forth in SEQ ID NOs: 4, 5, and 6, respectively;
2) the heavy chain variable region CDR1, the heavy chain variable region CDR2, and the heavy chain variable region CDR3 as set forth in SEQ ID NOs: 1, 2, and 75, respectively; and the light chain variable region CDR1, the light chain variable region CDR2, and the light chain variable region CDR3 as set forth in SEQ ID NOs: 4, 5, and 6, respectively;
3) the heavy chain variable region CDR1, the heavy chain variable region CDR2, and the heavy chain variable region CDR3 as set forth in SEQ ID NOs: 1, 2, and 76, respectively; and the light chain variable region CDR1, the light chain variable region CDR2, and the light chain variable region CDR3 as set forth in SEQ ID NOs: 4, 5, and 6, respectively;
4) the heavy chain variable region CDR1, the heavy chain variable region CDR2, and the heavy chain variable region CDR3 as set forth in SEQ ID NOs: 1, 2, and 77, respectively; and the light chain variable region CDR1, the light chain variable region CDR2, and the light chain variable region CDR3 as set forth in SEQ ID NOs: 4, 5, and 6, respectively;
5) the heavy chain variable region CDR1, the heavy chain variable region CDR2, and the heavy chain variable region CDR3 as set forth in SEQ ID NOs: 1, 2, and 78, respectively; and the light chain variable region CDR1, the light chain variable region CDR2, and the light chain variable region CDR3 as set forth in SEQ ID NOs: 4, 5, and 6, respectively;
6) the heavy chain variable region CDR1, the heavy chain variable region CDR2, and the heavy chain variable region CDR3 as set forth in SEQ ID NOs: 1, 2, and 79, respectively; and the light chain variable region CDR1, the light chain variable region CDR2, and the light chain variable region CDR3 as set forth in SEQ ID NOs: 4, 5, and 6, respectively;
7) the heavy chain variable region CDR1, the heavy chain variable region CDR2, and the heavy chain variable region CDR3 as set forth in SEQ ID NOs: 1, 2, and 80, respectively; and the light chain variable region CDR1, the light chain variable region CDR2, and the light chain variable region CDR3 as set forth in SEQ ID NOs: 4, 5, and 6, respectively;
8) the heavy chain variable region CDR1, the heavy chain variable region CDR2, and the heavy chain variable region CDR3 as set forth in SEQ ID NOs: 1, 2, and 81, respectively; and the light chain variable region CDR1, the light chain variable region CDR2, and the light chain variable region CDR3 as set forth in SEQ ID NOs: 4, 5, and 6, respectively;
9) the heavy chain variable region CDR1, the heavy chain variable region CDR2, and the heavy chain variable region CDR3 as set forth in SEQ ID NOs: 1, 2, and 82, respectively; and the light chain variable region CDR1, the light chain variable region CDR2, and the light chain variable region CDR3 as set forth in SEQ ID NOs: 4, 5, and 6, respectively;
10) the heavy chain variable region CDR1, the heavy chain variable region CDR2, and the heavy chain variable region CDR3 as set forth in SEQ ID NOs: 1, 2, and 83, respectively; and the light chain variable region CDR1, the light chain variable region CDR2, and the light chain variable region CDR3 as set forth in SEQ ID NOs: 4, 5, and 6, respectively;
11) the heavy chain variable region CDR1, the heavy chain variable region CDR2, and the heavy chain variable region CDR3 as set forth in SEQ ID NOs: 1, 2, and 3, respectively; and the light chain variable region CDR1, the light chain variable region CDR2, and the light chain variable region CDR3 as set forth in SEQ ID NOs: 4, 5, and 6, respectively;
12) the heavy chain variable region CDR1, the heavy chain variable region CDR2, and the heavy chain variable region CDR3 as set forth in SEQ ID NOs: 1, 2, and 7, respectively; and the light chain variable region CDR1, the light chain variable region CDR2, and the light chain variable region CDR3 as set forth in SEQ ID NOs: 4, 5, and 6, respectively;
13) the heavy chain variable region CDR1, the heavy chain variable region CDR2, and the heavy chain variable region CDR3 as set forth in SEQ ID NOs: 1, 2, and 8, respectively; and the light chain variable region CDR1, the light chain variable region CDR2, and the light chain variable region CDR3 as set forth in SEQ ID NOs: 4, 5, and 87, respectively;
14) the heavy chain variable region CDR1, the heavy chain variable region CDR2, and the heavy chain variable region CDR3 as set forth in SEQ ID NOs: 1, 2, and 8, respectively; and the light chain variable region CDR1, the light chain variable region CDR2, and the light chain variable region CDR3 as set forth in SEQ ID NOs: 4, 5, and 88, respectively;
15) the heavy chain variable region CDR1, the heavy chain variable region CDR2, and the heavy chain variable region CDR3 as set forth in SEQ ID NOs: 1, 2, and 8, respectively; and the light chain variable region CDR1, the light chain variable region CDR2, and the light chain variable region CDR3 as set forth in SEQ ID NOs: 4, 5, and 9, respectively;
16) the heavy chain variable region CDR1, the heavy chain variable region CDR2, and the heavy chain variable region CDR3 as set forth in SEQ ID NOs: 1, 2, and 84, respectively; and the light chain variable region CDR1, the light chain variable region CDR2, and the light chain variable region CDR3 as set forth in SEQ ID NOs: 4, 5, and 89, respectively;
17) the heavy chain variable region CDR1, the heavy chain variable region CDR2, and the heavy chain variable region CDR3 as set forth in SEQ ID NOs: 1, 2, and 85, respectively; and the light chain variable region CDR1, the light chain variable region CDR2, and the light chain variable region CDR3 as set forth in SEQ ID NOs: 4, 5, and 90, respectively;
18) the heavy chain variable region CDR1, the heavy chain variable region CDR2, and the heavy chain variable region CDR3 as set forth in SEQ ID NOs: 1, 2, and 86, respectively; and the light chain variable region CDR1, the light chain variable region CDR2, and the light chain variable region CDR3 as set forth in SEQ ID NOs: 4, 5, and 92, respectively; or
19) the heavy chain variable region CDR1, the heavy chain variable region CDR2, and the heavy chain variable region CDR3 as set forth in SEQ ID NOs: 1, 2 and 8, respectively; and the light chain variable region CDR1, the light chain variable region CDR2, and the light chain variable region CDR3 as set forth in SEQ ID NOs: 4, 5 and 91, respectively.

5. The antibody or antigen binding fragment thereof according to any one of claims 1 to 4, comprising a heavy chain framework region and/or a light chain framework region.

6. The antibody or antigen binding fragment thereof according to claim 5, wherein at least a portion of the heavy chain framework region and/or the light chain framework region is from at least one of a murine antibody, a human antibody, a primate antibody, a bovine antibody, an equine antibody, a dairy bovine antibody, a porcine antibody, an ovine antibody, a caprine antibody, a canine antibody, a feline antibody, a rabbit-derived antibody, a camelid antibody, donkey-derived antibody, a deer-derived antibody, a mink-derived antibody, a chicken-derived antibody, a duck-derived antibody, a goose-derived antibody, a turkey-derived antibody, gamecock-derived antibody, or a mutant thereof, and preferably, at least one of a murine antibody, a human antibody, or a primate antibody.

7. The antibody or antigen binding fragment thereof according to claim 5, comprising:
a heavy chain variable region having an amino acid sequence as set forth in one of SEQ ID NOs: 10, 12 to 13, 15 to 24, 27, and 31, or a conservative modification form thereof; and/or
a light chain variable region having an amino acid sequence as set forth in one of SEQ ID NOs: 11, 14, 25 to 26, 28, and 30, or a conservative modification form thereof.

8. The antibody or antigen binding fragment thereof according to claim 5, comprising:
a heavy chain variable region having an amino acid sequence with at least 90% sequence identity to an amino acid sequence as set forth in one of SEQ ID NOs: 10, 12 to 13, 15 to 24, 27, and 31; and/or
a light chain variable region having an amino acid sequence with at least 90% sequence identity to an amino acid sequence as set forth in one of SEQ ID NOs: 11, 14, 25 to 26, 28, and 30.

9. The antibody or antigen binding fragment thereof according to claim 8, comprising amino acid sequences as set forth in a combination of the heavy chain variable region and the light chain variable region in any one of the following groups, or amino acid sequences having at least 90% sequence identity thereto:
| Group number of the antibody or antigen binding fragment | Heavy chain variable region | Light chain variable region |
|---|---|---|
| 1 | SEQ ID NO: 15 | SEQ ID NO: 11 |
| 2 | SEQ ID NO: 16 | SEQ ID NO: 11 |
| 3 | SEQ ID NO: 17 | SEQ ID NO: 11 |
| 4 | SEQ ID NO: 17 | SEQ ID NO: 11 |
| 5 | SEQ ID NO: 19 | SEQ ID NO: 11 |
| 6 | SEQ ID NO: 20 | SEQ ID NO: 11 |
| 7 | SEQ ID NO: 21 | SEQ ID NO: 11 |
| 8 | SEQ ID NO: 22 | SEQ ID NO: 11 |
| 9 | SEQ ID NO: 23 | SEQ ID NO: 11 |
| 10 | SEQ ID NO: 24 | SEQ ID NO: 11 |
| 11 | SEQ ID NO: 10 | SEQ ID NO: 11 |
| 12 | SEQ ID NO: 12 | SEQ ID NO: 11 |
| 13 | SEQ ID NO: 13 | SEQ ID NO: 25 |
| 14 | SEQ ID NO: 13 | SEQ ID NO: 26 |
| 15 | SEQ ID NO: 13 | SEQ ID NO: 14 |
| 16 | SEQ ID NO: 27 | SEQ ID NO: 28 |
| 17 | SEQ ID NO: 29 | SEQ ID NO: 28 |
| 18 | SEQ ID NO: 13 | SEQ ID NO: 30 |
| 19 | SEQ ID NO: 31 | SEQ ID NO: 26 |

10. The antibody or antigen binding fragment thereof according to any one of claims 1 to 4, further comprising a constant region, wherein:
the constant region comprises at least one of a heavy chain constant region or a light chain constant region.

11. The antibody or antigen binding fragment thereof according to claim 10, wherein at least a portion of at least one of the heavy chain constant region or the light chain constant region is derived from at least one of a murine antibody, a human antibody, a primate antibody, a bovine antibody, an equine antibody, a dairy bovine antibody, a porcine antibody, an ovine antibody, a caprine antibody, a canine antibody, a feline antibody, a rabbit-derived antibody, a camelid antibody, a donkey-derived antibody, a deer-derived antibody, a mink-derived antibody, a chicken-derived antibody, a duck-derived antibody, a goose-derived antibody, a turkey-derived antibody, a gamecock-derived antibody, or a mutant thereof.

12. The antibody or antigen binding fragment thereof according to claim 10, wherein:
the heavy chain constant region comprises a heavy chain constant region selected from IgG1, IgG2, IgG3, IgG4, IgA, IgM, IgE, or IgD; and/or
the light chain constant region comprises a light chain constant region selected from a κ-type light chain constant region or a λ-type light chain constant region.

13. The antibody or antigen binding fragment thereof according to claim 11, wherein the light chain constant region and the heavy chain constant region are both derived from a murine antibody or a mutant thereof; or a human antibody or a mutant thereof.

14. The antibody or antigen binding fragment thereof according to claim 10, wherein:
the N-terminus of the heavy chain constant region is linked to the C-terminus of the heavy chain variable region; and/or
the N-terminus of the light chain constant region is linked to the N-terminus of the light chain variable region.

15. The antibody or antigen binding fragment thereof according to claim 10, wherein:
the heavy chain constant region has an amino acid sequence as set forth in SEQ ID NO: 93 or an amino acid sequence having at least 80% identity thereto; and/or
the light chain constant region has an amino acid sequence as set forth in SEQ ID NO: 94 or an amino acid sequence having at least 80% identity thereto.

16. The antibody or antigen binding fragment thereof according to claim 10, wherein:
the C-terminus of the heavy chain variable region is linked to the N-terminus of the light chain variable region, or
the N-terminus of the heavy chain variable region is linked to the C-terminus of the light chain variable region.

17. The antibody or antigen binding fragment thereof according to claim 16, further comprising a first linking peptide, wherein:
the C-terminus of the heavy chain variable region is linked to the N-terminus of the first linking peptide, and the C-terminus of the first linking peptide is linked to the N-terminus of the light chain variable region, or
the C-terminus of the light chain variable region is linked to the N-terminus of the first linking peptide, and the C-terminus of the first linking peptide is linked to the N-terminus of the heavy chain variable region.

18. The antibody or antigen binding fragment thereof according to any one of claims 1 to 4, wherein:
the antibody comprises at least one of a full-length monoclonal antibody, chimeric antibody, humanized antibody, Fv, scFv, Fab, Fab', Fab'-SH, or F(ab')₂; and
the antigen binding fragment of the antibody comprises at least one of a F(ab')₂ fragment, Fab' fragment, Fab fragment, F(ab)₂ fragment, Fv fragment, scFv fragment, scFv-Fc fusion protein, scFv-Fv fusion protein, or a minimum recognition unit.

19. The antibody or antigen binding fragment thereof according to claim 18, comprising:
a heavy chain having an amino acid sequence as set forth in one of SEQ ID NOs: 32, 34, 35, and 37 to 46, or an amino acid sequence having at least 80% identity thereto; and/or
a light chain having an amino acid sequence as set forth in one of SEQ ID NOs: 33, 36, and 47 to 48, or an amino acid sequence having at least 80% identity thereto.

20. The antibody or antigen binding fragment thereof according to claim 18, wherein the antibody or antigen binding fragment thereof has amino acid sequences as set forth in a combination of a heavy chain and a light chain in any one of the following groups, or amino acid sequences having at least 80% sequence identity thereto:
| Group number of the antibody or antigen binding fragment thereof | Heavy chain | Light chain |
|---|---|---|
| 1 | SEQ ID NO: 37 | SEQ ID NO: 33 |
| 2 | SEQ ID NO: 38 | SEQ ID NO: 33 |
| 3 | SEQ ID NO: 39 | SEQ ID NO: 33 |
| 4 | SEQ ID NO: 40 | SEQ ID NO: 33 |
| 5 | SEQ ID NO: 41 | SEQ ID NO: 33 |
| 6 | SEQ ID NO: 42 | SEQ ID NO: 33 |
| 7 | SEQ ID NO: 43 | SEQ ID NO: 33 |
| 8 | SEQ ID NO: 44 | SEQ ID NO: 33 |
| 9 | SEQ ID NO: 45 | SEQ ID NO: 33 |
| 10 | SEQ ID NO: 46 | SEQ ID NO: 33 |
| 11 | SEQ ID NO: 32 | SEQ ID NO: 33 |
| 12 | SEQ ID NO: 34 | SEQ ID NO: 33 |
| 13 | SEQ ID NO: 35 | SEQ ID NO: 47 |
| 14 | SEQ ID NO: 35 | SEQ ID NO: 48 |
| 15 | SEQ ID NO: 35 | SEQ ID NO: 36 |
| 16 | SEQ ID NO: 95 | SEQ ID NO: 119 |
| 17 | SEQ ID NO: 96 | SEQ ID NO: 120 |
| 18 | SEQ ID NO: 97 | SEQ ID NO: 121 |
| 19 | SEQ ID NO: 98 | SEQ ID NO: 122 |

21. The antibody or antigen binding fragment thereof according to claim 18, having an amino acid sequence as set forth in any one of SEQ ID NOs: 99 to 117, or an amino acid sequence having at least 80% sequence identity thereto.

22. The antibody or antigen binding fragment thereof according to claim 18, having amino acid sequences as set forth in a combination of Fab VL-CL and Fab VH-CH1 in any one of the following groups, or amino acid sequences having at least 80% sequence identity thereto:
| Group number of the antibody or antigen binding fragment | Fab VH-CH1 | Fab VL-CL |
|---|---|---|
| 1 | SEQ ID NO: 123 | SEQ ID NO: 33 |
| 2 | SEQ ID NO: 124 | SEQ ID NO: 33 |
| 3 | SEQ ID NO: 125 | SEQ ID NO: 33 |
| 4 | SEQ ID NO: 126 | SEQ ID NO: 33 |
| 5 | SEQ ID NO: 127 | SEQ ID NO: 33 |
| 6 | SEQ ID NO: 128 | SEQ ID NO: 33 |
| 7 | SEQ ID NO: 129 | SEQ ID NO: 33 |
| 8 | SEQ ID NO: 130 | SEQ ID NO: 33 |
| 9 | SEQ ID NO: 131 | SEQ ID NO: 33 |
| 10 | SEQ ID NO: 132 | SEQ ID NO: 33 |
| 11 | SEQ ID NO: 133 | SEQ ID NO: 33 |
| 12 | SEQ ID NO: 134 | SEQ ID NO: 33 |
| 13 | SEQ ID NO: 135 | SEQ ID NO: 47 |
| 14 | SEQ ID NO: 136 | SEQ ID NO: 36 |
| 15 | SEQ ID NO: 137 | SEQ ID NO: 48 |
| 16 | SEQ ID NO: 138 | SEQ ID NO: 119 |
| 17 | SEQ ID NO: 139 | SEQ ID NO: 120 |
| 18 | SEQ ID NO: 140 | SEQ ID NO: 121 |
| 19 | SEQ ID NO: 141 | SEQ ID NO: 122 |

23. The antibody or antigen binding fragment thereof according to claim 18, wherein the antibody or antigen binding fragment thereof has an amino acid sequence as set forth in any one of SEQ ID NOs: 142 to 160, or an amino acid sequence having at least 80% sequence identity thereto.

24. A multi-specific antibody, comprising:
a first antigen binding region, wherein the first antigen binding region comprises the anti-CD3 antibody or antigen binding fragment thereof according to any one of claims 1 to 23; and
a second antigen binding region, wherein the second antigen binding region has biomolecule binding activity, the biomolecule not being CD3.

25. The multi-specific antibody according to claim 24, wherein the multi-specific antibody is selected from one of a bispecific antibody, a trispecific antibody, or a tetraspecific antibody, and preferably, the multi-specific antibody is a bispecific antibody.

26. The multi-specific antibody according to claim 25, wherein the bispecific antibody comprises a symmetric bispecific antibody or an asymmetric bispecific antibody.

27. The multi-specific antibody according to claim 24, wherein the second antigen binding region is a binding protein of the biomolecule or a fragment thereof.

28. The multi-specific antibody according to claim 24, wherein the biomolecule is selected from at least one of PDL1, CD47, TIGIT, CD73, CD33, CEACAM1, CEACAM5, CEACAM6, STING, WNT, Beta catenin, B7H3, VISITA, CD19, BCMA, CD22, CD20, CD123, CD38, CEA, CD25, CD46, CD138, PSCA, PSMA, PSA, MUC1, MUC16, NY-ESO-1, GD2, WT1, Mesothelin, MAGE-A3, GPC3, PRAME, Globo H, AFP, Trop2, FOLR1, SP, Sca-1, CD133, or EPCAM.

29. The multi-specific antibody according to claim 27, wherein the binding protein or fragment thereof is selected from at least one of an antibody or antigen binding fragment thereof, a receptor, or a ligand.

30. The multi-specific antibody according to claim 29, wherein the bispecific antibody is an asymmetric bispecific antibody.

31. The multi-specific antibody according to claim 24, wherein the first antigen binding region comprises a first scFv fragment, a first Fv fragment, a first Fab fragment, or a first Fab-Linker fragment.

32. The multi-specific antibody according to claim 31, wherein the first antigen binding region further comprises a first Fc peptide segment.

33. The multi-specific antibody according to claim 32, wherein:
the N-terminus of the first Fc peptide segment is linked to the C-terminus of the scFv fragment or the C-terminus of the Fv fragment; or
the N-terminus of the first Fc peptide segment is linked to the C-terminus of CH1 of the Fab fragment or the C-terminus of the Fab-Linker fragment.

34. The multi-specific antibody according to claim 32, wherein the first antigen binding region further comprises a second linking peptide, wherein:
the N-terminus of the first Fc peptide segment is linked to the C-terminus of the second linking peptide, and the N-terminus of the second linking peptide is linked to the C-terminus of the scFv fragment or the C-terminus of the Fv fragment; or
the N-terminus of the first Fc peptide segment is linked to the C-terminus of the second linking peptide, and the N-terminus of the second linking peptide is linked to the C-terminus of CH1 of the Fab fragment or the C-terminus of the Fab-Linker fragment.

35. The multi-specific antibody according to claim 32, wherein the first Fc peptide segment is selected from a human Fc peptide segment.

36. The multi-specific antibody according to claim 35, wherein the first Fc peptide segment is a human IgG1 Fc peptide segment.

37. The multi-specific antibody according to claim 32, wherein:
the first Fc peptide segment has an amino acid sequence as set forth in SEQ ID NO: 118; or
the first Fc peptide segment has an amino acid sequence as set forth in SEQ ID NO: 166.

38. The multi-specific antibody according to claim 31, wherein the first scFv fragment has an amino acid sequence as set forth in any one of SEQ ID NOs: 95 to 117.

39. The multi-specific antibody according to claim 31, wherein the first Fab fragment has amino acid sequences as set forth in a combination of Fab VL-CL and Fab VH-CH1 in any one of the following groups:
| Group number of the first Fab fragment | Fab VH-CH1 | Fab VL-CL |
|---|---|---|
| 1 | SEQ ID NO: 123 | SEQ ID NO: 33 |
| 2 | SEQ ID NO: 124 | SEQ ID NO: 33 |
| 3 | SEQ ID NO: 125 | SEQ ID NO: 33 |
| 4 | SEQ ID NO: 126 | SEQ ID NO: 33 |
| 5 | SEQ ID NO: 127 | SEQ ID NO: 33 |
| 6 | SEQ ID NO: 128 | SEQ ID NO: 33 |
| 7 | SEQ ID NO: 129 | SEQ ID NO: 33 |
| 8 | SEQ ID NO: 130 | SEQ ID NO: 33 |
| 9 | SEQ ID NO: 131 | SEQ ID NO: 33 |
| 10 | SEQ ID NO: 132 | SEQ ID NO: 33 |
| 11 | SEQ ID NO: 133 | SEQ ID NO: 33 |
| 12 | SEQ ID NO: 134 | SEQ ID NO: 33 |
| 13 | SEQ ID NO: 135 | SEQ ID NO: 47 |
| 14 | SEQ ID NO: 136 | SEQ ID NO: 36 |
| 15 | SEQ ID NO: 137 | SEQ ID NO: 48 |
| 16 | SEQ ID NO: 138 | SEQ ID NO: 119 |
| 17 | SEQ ID NO: 139 | SEQ ID NO: 120 |
| 18 | SEQ ID NO: 140 | SEQ ID NO: 121 |
| 19 | SEQ ID NO: 141 | SEQ ID NO: 122 |

40. The multi-specific antibody according to claim 31, wherein the first Fab-Linker has an amino acid sequence as set forth in any one of SEQ ID NOs: 142 to 160.

41. The multi-specific antibody according to claim 24, wherein the first antigen binding region has an amino acid sequence as set forth in any one of SEQ ID NOs: 49 to 52 and 54 to 68.

42. The multi-specific antibody according to claim 28, wherein the biomolecule is Trop2 or PDL1.

43. The multi-specific antibody according to claim 42, wherein the second antigen binding region comprises:
a first anti-Trop2 antibody or antigen binding fragment thereof; or
a first anti-PDL1 antibody or an antigen binding fragment thereof.

44. The multi-specific antibody according to claim 43, wherein:
the first anti-Trop2 antibody or antigen binding fragment thereof is a second scFab fragment or a second Fab fragment of anti-Trop2; or
the first anti-PDL1 antibody or antigen binding fragment thereof is a third scFab fragment or a third Fab fragment of anti-PDL1.

45. The multi-specific antibody according to claim 44, wherein:
the second scFab fragment has an amino acid sequence as set forth in SEQ ID NO: 161;
the second Fab fragment has an amino acid sequence as set forth in SEQ ID NO: 162 and 70;
the third scFab fragment has an amino acid sequence as set forth in SEQ ID NO: 163; or
the third Fab fragment has an amino acid sequence as set forth in SEQ ID NO: 164 and 72.

46. The multi-specific antibody according to claim 44, wherein the second antigen binding region further comprises a second Fc fragment, wherein:
the N-terminus of the second Fc fragment is linked to the C-terminus of CH1 of the second scFab fragment, the C-terminus of CH1 of the second Fab fragment, the C-terminus of CH1 of the third scFab fragment, or the C-terminus of CH1 of the third Fab fragment.

47. The multi-specific antibody according to claim 46, wherein the second Fc peptide segment is selected from a human Fc peptide segment.

48. The multi-specific antibody according to claim 47, wherein the second Fc peptide segment is a human IgG1 Fc peptide segment.

49. The multi-specific antibody according to claim 46, wherein the second Fc peptide segment has an amino acid sequence as set forth in SEQ ID NO: 165.

50. The multi-specific antibody according to claim 46, wherein the first Fc peptide segment and the second Fc peptide segment are linked via a knob-into-hole structure.

51. The multi-specific antibody according to claim 43, wherein:
the second antigen binding region has an amino acid sequence as set forth in SEQ ID NO: 161 or 163;
the second antigen binding region has an amino acid sequence as set forth in SEQ ID NO: 162 and 70; or
the second antigen binding region has an amino acid sequence as set forth in SEQ ID NO: 164 and 72.

52. A conjugate, comprising:
the antibody or antigen binding fragment thereof according to any one of claims 1 to 23, or the multi-specific antibody according to any one of claims 24 to 51; and
a conjugated moiety, the conjugated moiety being linked to the antibody or antigen binding fragment thereof or the multi-specific antibody.

53. The conjugate according to claim 52, wherein the conjugated moiety comprises at least one selected from a carrier, a medicament, a toxin, a cytokine, a protein tag, a modifier, a therapeutic agent, or a chemotherapeutic agent.

54. A nucleic acid, encoding the antibody or antigen binding fragment thereof according to any one of claims 1 to 23, or the multi-specific antibody according to any one of claims 24 to 51.

55. A vector or transformant, comprising the nucleic acid according to claim 54.

56. The vector or transformant according to claim 55, wherein the vector is a eukaryotic vector or prokaryotic vector.

57. The vector or transformant according to claim 55, wherein the vector comprises at least one selected from a plasmid vector, an adenovirus vector, a lentivirus vector, or an adeno-associated virus vector.

58. A cell, carrying the nucleic acid according to claim 54, or the vector or transformant according to any one of claims 55 to 57; or expressing the antibody or antigen binding fragment thereof according to any one of claims 1 to 23 or the multi-specific antibody according to any one of claims 24 to 51.

59. The cell according to claim 58, wherein the cell is a prokaryotic cell, a eukaryotic cell, or a bacteriophage.

60. A pharmaceutical composition, comprising:
the antibody or antigen binding fragment thereof according to any one of claims 1 to 23;
the multi-specific antibody according to any one of claims 24 to 51;
the conjugate according to any one of claims 52 to 53;
the nucleic acid according to claim 54; or
the vector or transformant according to any one of claims 55 to 57.

61. The pharmaceutical composition according to claim 60, further comprising a pharmaceutically acceptable adjuvant.

62. The pharmaceutical composition according to claim 61, wherein the adjuvant comprises one or more pharmaceutically acceptable excipients, diluents, stabilizers, or carriers.

63. The pharmaceutical composition according to claim 60, wherein the pharmaceutical composition is an injection.

64. A kit, comprising:
the antibody or antigen binding fragment thereof according to any one of claims 1 to 23; or
the multi-specific antibody according to any one of claims 24 to 51.

65. Use of the antibody or antigen binding fragment thereof according to any one of claims 1 to 23 or the multi-specific antibody according to any one of claims 24 to 51 in the manufacture of a kit for detecting CD3.

66. Use of the antibody or antigen binding fragment thereof according to any one of claims 1 to 23, the multi-specific antibody according to any one of claims 24 to 51, the conjugate according to any one of claims 52 to 53, the nucleic acid according to claim 54, the vector or transformant according to any one of claims 55 to 57, or the pharmaceutical composition according to any one of claims 60 to 63 in the manufacture of a medicament for regulating an immune response and/or inhibiting tumor growth, or for the prevention and/or treatment of a CD3-related disease and/or cancer or tumor or infection or autoimmune disease.

67. The use according to claim 66, wherein the cancer or tumor or infection or autoimmune disease is a Trop2- or PDL1-related disease.

68. The use according to claim 67, wherein:
the cancer or tumor is at least one of lung cancer, liver cancer, ovarian cancer, cervical cancer, skin cancer, bladder cancer, colon cancer, rectal cancer, breast cancer, pancreatic cancer, prostate cancer, glioma, renal cancer, gastric cancer, esophageal cancer, oral squamous cell carcinoma, or head-and-neck cancer; and/or
the cancer or tumor is at least one of non-small cell lung cancer, malignant melanoma, renal cell carcinoma, head-and-neck squamous cell carcinoma, urothelial carcinoma, colorectal cancer, liver cancer, or classical Hodgkin's lymphoma.

69. A method for regulating an immune response and/or inhibiting tumor growth or preventing and/or treating a CD3-related disease and/or cancer, tumor, infection, or autoimmune disease, comprising:
administering to a subject a pharmaceutically acceptable amount of the antibody or antigen binding fragment thereof according to any one of claims 1 to 23, the multi-specific antibody according to any one of claims 24 to 51, the conjugate according to any one of claims 52 to 53, the nucleic acid according to claim 54, the vector or transformant according to any one of claims 55 to 57, or the pharmaceutical composition according to any one of claims 60 to 63.

70. The method according to claim 69, wherein the cancer or tumor or infection or autoimmune disease is a Trop2- or PDL1-related disease.

71. The method according to claim 70, wherein:
the cancer or tumor is at least one of lung cancer, liver cancer, ovarian cancer, cervical cancer, skin cancer, bladder cancer, colon cancer, rectal cancer, breast cancer, pancreatic cancer, prostate cancer, glioma, renal cancer, gastric cancer, esophageal cancer, oral squamous cell carcinoma, or head-and-neck cancer; and/or
the cancer or tumor is at least one of non-small cell lung cancer, malignant melanoma, renal cell carcinoma, head-and-neck squamous cell carcinoma, urothelial carcinoma, colorectal cancer, liver cancer, or classical Hodgkin's lymphoma.
